# EUROPEAN PATENT SPECIFICATION

(11) **EP 4 213 935 B1**
(45) Date of publication and mention of the grant of the patent: **04.11.2026**
(21) Application number: 21867984.3
(22) Date of filing: 15.09.2021
(51) Int. Cl.: A61N 5/10, A61B 34/20, A61B 5/00, A61B 6/03

(54) **TECHNIQUES FOR DETECTING MOVEMENT DURING RADIOTHERAPY TREATMENT**
VERFAHREN ZUR ERKENNUNG VON BEWEGUNG WÄHREND EINER STRAHLENTHERAPIEBEHANDLUNG
TECHNIQUES DE DÉTECTION DE MOUVEMENT PENDANT UN TRAITEMENT PAR RADIOTHÉRAPIE

(30) Priority: 16.09.2020 US 202062706893 P
(43) Date of publication of application: 26.07.2023
(73) Proprietor: Elekta Limited, Montreal, Québec H3A 2J5 (CA)
(72) Inventor: LACHAINE, Martin Emile, Montreal, Québec H3A 2J5 (CA); FALCO, Tony, Montreal, Québec H3A 2J5 (CA)
(74) Representative: Hamer, Thomas Daniel
(86) International application number: PCT/CA2021/051287
(87) International publication number: WO 2022/056630

(56) References cited:
- WO-A1-2013/134597
- WO-A1-2020/028422
- US-A1- 2015 217 139
- US-A1- 2017 360 325
- US-A1- 2018 050 222
- US-A1- 2018 243 586
- US-A1- 2019 030 370
- US-A1- 2020 129 780
- US-A1- 2020 129 784
- US-A1- 2020 160 972
- US-B1- 6 345 114
- HUSSEIN M ET AL: "Challenges in calculation of the gamma index in radiotherapy - Towards good practice", PHYSICA MEDICA, ACTA MEDICA EDIZIONI E CONGRESSI, ROME, IT, vol. 36, 14 March 2017 (2017-03-14), pages 1 - 11, XP029977175, ISSN: 1120-1797, DOI: 10.1016/J.EJMP.2017.03.001

## Description

### CLAIM OF PRIORITY

This application claims the benefit of priority of U.S. Provisional Patent Application Serial Number 62/706,893, titled "MV IMAGING FOR ONLINE ADAPTIVE RADIOTHERAPY" to Martin Emile Lachaine et al., filed on September 16, 2020.

### TECHNICAL FIELD

Embodiments of the present disclosure pertain generally to radiotherapy treatment sessions and specifically to imaging techniques.

### BACKGROUND

Radiation therapy (or "radiotherapy") can be used to treat cancers or other ailments in mammalian (e.g., human and animal) tissue. One such radiotherapy technique involves irradiation with a Gamma Knife, whereby a patient is irradiated by a large number of low-intensity gamma ray beams that converge with high intensity and high precision at a target (e.g., a tumor). In another embodiment, radiotherapy is provided using a linear accelerator, whereby a tumor is irradiated by high-energy particles (e.g., electrons, protons, ions, high-energy photons, and the like). The placement and dose of the radiation beam must be accurately controlled to ensure the tumor receives the prescribed radiation, and the placement of the beam should be such as to minimize damage to the surrounding healthy tissue, often called the organ(s) at risk (OARs). Radiation is termed "prescribed" because a physician orders a predefined amount of radiation to the tumor and surrounding organs similar to a prescription for medicine. Generally, ionizing radiation in the form of a collimated beam is directed from an external radiation source toward a patient.

A specified or selectable beam energy can be used, such as for delivering a diagnostic energy level range or a therapeutic energy level range. Modulation of a radiation beam can be provided by one or more attenuators or collimators (e.g., a multi-leaf collimator (MLC)). The intensity and shape of the radiation beam can be adjusted by collimation to avoid damaging healthy tissue (e.g., OARs) adjacent to the targeted tissue by conforming the projected beam to a profile of the targeted tissue.

The treatment planning procedure may include using a three-dimensional (3D) image of the patient to identify a target region (e.g., the tumor) and to identify critical organs near the tumor. Creation of a treatment plan can be a time-consuming process where a planner tries to comply with various treatment objectives or constraints (e.g., dose volume histogram (DVH), overlap volume histogram (OVH)), taking into account their individual importance (e.g., weighting) in order to produce a treatment plan that is clinically acceptable. This task can be a time-consuming trial-and-error process that is complicated by the various OARs because as the number of OARs increases (e.g., up to thirteen for a head-and-neck treatment), so does the complexity of the process. OARs distant from a tumor may be easily spared from radiation, while OARs close to or overlapping a target tumor may be difficult to spare.

Traditionally, for each patient, the initial treatment plan can be generated in an "offline" manner. The treatment plan can be developed well before radiation therapy is delivered, such as using one or more medical imaging techniques. Imaging information can include, for example, images from X-rays, computed tomography (CT), nuclear magnetic resonance (MR), positron emission tomography (PET), single-photon emission computed tomography (SPECT), or ultrasound. A health care provider, such as a physician, may use 3D imaging information indicative of the patient anatomy to identify one or more target tumors along with the OARs near the tumor(s). The health care provider can delineate the target tumor that is to receive a prescribed radiation dose using a manual technique, and the health care provider can similarly delineate nearby tissue, such as organs, at risk of damage from the radiation treatment. Alternatively, or additionally, an automated tool (e.g., ABAS provided by Elekta AB, Sweden) can be used to assist in identifying or delineating the target tumor and organs at risk. A radiation therapy treatment plan ("treatment plan") can then be created using an optimization technique based on clinical and dosimetric objectives and constraints (e.g., the maximum, minimum, and fraction of dose of radiation to a fraction of the tumor volume ("95% of target shall receive no less than 100% of prescribed dose"), and like measures for the critical organs). The optimized plan is comprised of numerical parameters that specify the direction, cross-sectional shape, and intensity of each radiation beam.

The treatment plan can then be later executed by positioning the patient in the treatment machine and delivering the prescribed radiation therapy directed by the optimized plan parameters. The radiation therapy treatment plan can include dose "fractioning," whereby a sequence of radiation treatments is provided over a predetermined period of time (e.g., 30-45 daily fractions), with each treatment including a specified fraction of a total prescribed dose. However, during treatment, the position of the patient and the position of the target tumor in relation to the treatment machine (e.g., linear accelerator - "linac") is very important in order to ensure the target tumor and not healthy tissue is irradiated.

Since most patients receive more than one fraction of radiation as part of a course of therapy, and because the anatomy may change (deform) between these fractions, it is not straightforward to sum the doses delivered during the individual fractions so the physician can accurately gauge how the treatment is proceeding relative to the original intent as defined by the prescription.

US 6345114 B1 relates to a method and apparatus for calibration of radiation therapy equipment and verification of radiation treatment.

US 2018/050222 A1 relates to a method for tracking a target located within a body, whilst scanning the body using X-ray projections.

US 2020/129780 A1 relates to systems and techniques which may be used to estimate a patient state during a radiotherapy treatment.

### OVERVIEW

The invention is defined by the appended set of claims. This disclosure describes, among other things, techniques to simulate partial patient images, such as 2D Megavolt (MV) images or 2D CT images, such as slices or projections, as the patient representation evolves during the radiation treatment, and then compare one or more actual patient images obtained during the radiation treatment to the one or more simulated partial patient images. A resulting image similarity indication, such as a pass/fail signal, can then be provided to a radiation therapy system to represent movement of the organ occurring during the radiation treatment.

In some aspects, this disclosure is directed to a computer-implemented method for image-guided radiotherapy that can accommodate movement of an organ of a patient during radiation treatment, the computer-implemented method comprising: computing, using computer processor circuitry, one or more simulated partial images using a simulation model and simulation inputs including patient representation information and radiation treatment parameter information; comparing, using the computer processor circuitry, one or more actual images obtained during the radiation treatment, to the one or more simulated partial images; and generating, using the computer processor circuitry, a resulting image similarity indication to represent the movement of the organ occurring during the radiation treatment.

In some aspects, this disclosure is directed to a radiotherapy system, comprising: an image acquisition device configured to acquire images of an anatomical region of interest of a patient; a radiotherapy device configured to deliver a dose of radiation to the anatomical region of interest based on the images of the anatomical region of interest; and a processor device configured to: compute one or more simulated 2D Megavolt (MV) images or 2D diagnostic computed tomography (CT) images using a simulation model and simulation inputs including patient representation information and radiation treatment parameter information; compare one or more actual images obtained during the radiation treatment, to the one or more simulated images; and generate a resulting image similarity indication to represent the movement of the organ occurring during the radiation treatment.

### BRIEF DESCRIPTION OF THE DRAWINGS

In the drawings, which are not necessarily drawn to scale, like numerals describe substantially similar components throughout the several views. Like numerals having different letter suffixes represent different instances of substantially similar components. The drawings illustrate generally, by way of example but not by way of limitation, various embodiments discussed in the present document.
FIG. 1 illustrates an example of a radiotherapy system, according to some embodiments of the present disclosure.
FIG. 2A illustrates an example a radiation therapy system that can include radiation therapy output configured to provide a therapy beam, according to some embodiments of the present disclosure.
FIG. 2B illustrates an example of a system including a combined radiation therapy system and an imaging system, such as a cone beam computed tomography (CBCT) imaging system, according to some embodiments of the present disclosure.
FIG. 3 illustrates a partially cut-away view of an example system including a combined radiation therapy system and an imaging system, such as a nuclear MR imaging (MRI) system, according to some embodiments of the present disclosure.
FIGS. 4A and 4B depict the differences between an example MRI image and a corresponding CT image, respectively, according to some embodiments of the present disclosure.
FIG. 5 illustrates an example of a collimator configuration for shaping, directing, or modulating an intensity of a radiation therapy beam, according to some embodiments of the present disclosure.
FIG. 6 illustrates an example of a Gamma Knife radiation therapy system, according to some embodiments of the present disclosure.
FIG. 7 is a conceptual diagram illustrating a non-limiting example of a computer-implemented method for image-guided radiotherapy that can accommodate movement of an organ of a patient during radiation treatment using various techniques of this disclosure.
FIG. 8 is a conceptual diagram illustrating a non-limiting example of a computer-implemented method for image-guided radiotherapy that can accommodate movement of an organ of a patient during radiation treatment using various techniques of this disclosure.
FIG. 9 illustrates a flow diagram of an example of a computer-implemented method for image-guided radiotherapy that can accommodate movement of an organ of a patient during radiation treatment, according to some embodiments of the present disclosure.
FIG. 10 illustrates an example of a block diagram of a machine on which one or more of the methods as discussed herein can be implemented.

### DETAILED DESCRIPTION

In the following detailed description, reference is made to the accompanying drawings which form a part hereof, and which is shown by way of illustration-specific embodiments in which the present disclosure may be practiced. These embodiments, which are also referred to herein as "examples," are described in sufficient detail to enable those skilled in the art to practice the disclosure, and it is to be understood that the embodiments may be combined, or that other embodiments may be utilized, and that structural, logical, and electrical changes may be made without departing from the scope of the present disclosure. The following detailed description is, therefore, not be taken in a limiting sense, and the scope of the present disclosure is defined by the appended claims and their equivalents.

In online adaptive radiotherapy, it can be useful to have a three-dimensional (3D) representation of the patient while the patient is set up for radiation treatment delivery. For radiation treatment targets that move relatively slowly, such as for prostate treatments, for example, it can be sufficient to acquire a single 3D image before radiation treatment, to adapt the radiation treatment plan using this image, and to assume that the patient anatomy does not change. Alternatively, the radiation treatment can be broken down into multiple sub-fractions, such as to be delivered separately at different times, and each sub-fraction can be treated as a new fraction with a new image taken in advance to adapt the radiation treatment plan. This can help reduce the chance of missing significant motions occurring during a course of treatment that would otherwise not be compensated with an adapted radiation treatment plan.

For certain patients, such as those with a target organ or organ-at-risk that are heavily influenced by respiration, it can be desirable to dynamically adapt the radiation treatment plan to account for the impact of respiration on the patient's anatomy. To accomplish this, one approach can be to obtain 3D images at less than 500 millisecond intervals, preferably less than 100 millisecond intervals. It is currently not possible to acquire 3D images with such speeds without significant compromises in image quality.

To handle this, 3D images can be estimated during the treatment from fast partial data such as from surface data, two-dimensional (2D) kiloVolt (kV) projections, or 2D magnetic resonance (MR) slices. This can be accomplished with the aid of a model generated prior to beam-on, to form the evolving 3D image with sufficient speed. For example, see Elekta patent filings US2020129780, US20170360325, US20200129784, US20200160972, US Provisional Patent Application Serial No. 62/991,356 (SLW Ref. 4186.139PRV; Elekta Ref. 19US11PROV), and US Provisional Patent Application Serial No. 63/066,552 (SLW Ref. 4186.152PRV; Elekta Ref. 20US03). For another approach, see McClelland, Jamie R., et al. "A generalized framework unifying image registration and respiratory motion models and incorporating image reconstruction, for partial image data or full images," Physics in Medicine & Biology 62.11 (2017): 4273. The evolving images can be used to dynamically adjust the treatment plan, or they can be used for retrospective dose calculations, radiation therapy gating, or multileaf collimator (MLC) tracking applications.

Regardless of whether respiration is considered, it can be valuable to verify the underlying assumptions being used for the patient's 3D anatomy. For the slow-moving case, it can be helpful to validate whether the last 3D image of the patient used is still a good representation of the patient's anatomy. For cases in which respiration is considered, it is important to validate that the current estimate of the 3D image of the patient is accurate. The present inventors have recognized a need for a quick and efficient way to validate the 3D image of the patient at all times during treatment, with no additional radiation to the patient.

This disclosure describes, among other things, techniques to simulate partial patient images, such as 2D Megavolt (MV) images or 2D CT images, such as slices or projections, as the patient representation evolves during the radiation treatment, and then compare one or more actual patient images obtained during the radiation treatment to the one or more simulated partial patient images. A resulting image similarity indication, such as a pass/fail signal, can then be provided to a radiation therapy system to represent movement of the organ occurring during the radiation treatment.

For example, and as described in more detail below, an MV image can be simulated as accurately as possible, taking all of the imaging processes into account so that the simulated partial MV images can provide a near-exact match to the actual MV images. The simulated partial MV images can be compared to measured MV images throughout the treatment. If significant differences are observed, that can provide an indication that the patient representation is no longer representative of the actual patient anatomy. In such a case, the treatment can be interrupted, and a new patient representation can be generated before re-commencing the treatment. For example, a new 3D image can be acquired, or a new respiratory model can be generated that relates partial patient data to 3D image representations.

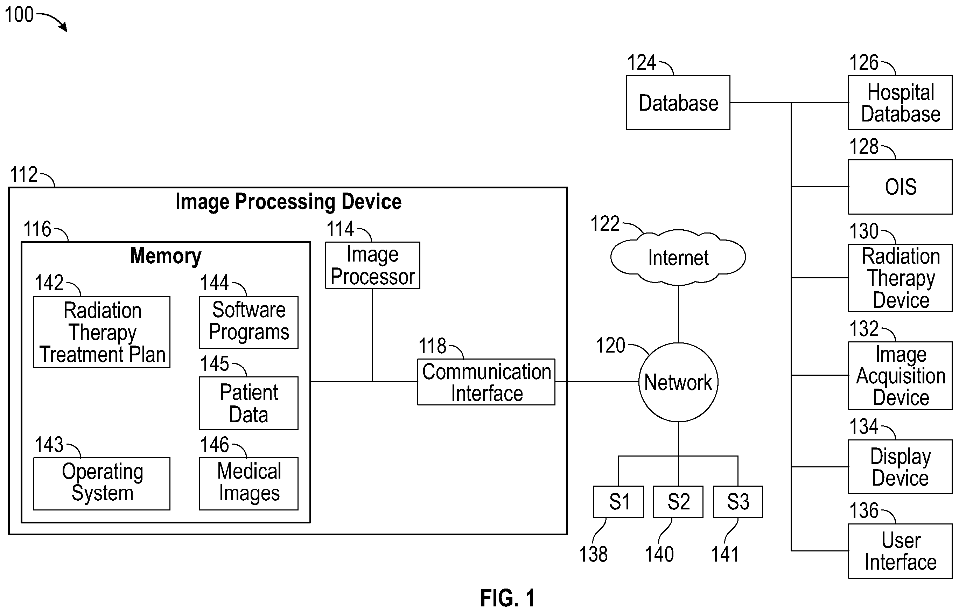

FIG. 1 illustrates an example of a radiotherapy system 100 for providing radiation therapy to a patient. The radiotherapy system 100 includes an image processing device 112. The image processing device 112 may be connected to a network 120. The network 120 may be connected to the Internet 122. The network 120 can connect the image processing device 112 with one or more of a database 124, a hospital database 126, an oncology information system (OIS) 128, a radiation therapy device 130, e.g., providing X-ray energy in a Mega electron-Volt (MeV) range, an image acquisition device 132, a display device 134, and a user interface 136. The image processing device 112 can be configured to generate radiation therapy treatment plans 142 to be used by the radiation therapy device 130. The radiation therapy device can also be referred to as an MV source in this disclosure.

The image processing device 112 may include a memory device 116, an image processor 114, and a communication interface 118. The memory device 116 may store computer-executable instructions, such as an operating system 143, radiation therapy treatment plans 142 (e.g., original treatment plans, adapted treatment plans and the like), software programs 144 (e.g., artificial intelligence, deep learning, neural networks, radiotherapy treatment plan software), and any other computer-executable instructions to be executed by the image processor 114.

In one embodiment, the software programs 144 may convert medical images of one format (e.g., MRI) to another format (e.g., CT) by producing synthetic images, such as pseudo-CT images. For instance, the software programs 144 may include image processing programs to train a predictive model for converting a medical image 146 in one modality (e.g., an MRI image) into a synthetic image of a different modality (e.g., a pseudo-CT image); alternatively, the trained predictive model may convert a CT image into an MRI image.

In another embodiment, the software programs 144 may register the patient image (e.g., a CT image or an MR image) with that patient's dose distribution (also represented as an image) so that corresponding image voxels and dose voxels are associated appropriately by the network.

In yet another embodiment, the software programs 144 may substitute functions of the patient images or processed versions of the images that emphasize some aspect of the image information. Such functions might emphasize edges or differences in voxel textures, or any other structural aspect useful to neural network learning.

In another embodiment, the software programs 144 may substitute functions of the dose distribution that emphasize some aspect of the dose information. Such functions might emphasize steep gradients around the target or any other structural aspect useful to neural network learning. The memory device 116 may store data, including medical images 146, patient data 145, and other data required to create and implement a radiation therapy treatment plan 142.

In yet another embodiment, the software programs 144 may generate a structural estimate (e.g., a 3D model of the region of interest) using an iterative image reconstruction process. The structural estimate may be or include an X-ray attenuation map that represents a 3D model of a region of interest. The structural estimate may be used to estimate or simulate X-ray measurements to be compared with real X-ray measurements for updating the structural estimate. Specifically, the software programs 144 can access a current structural estimate of the region of interest and generate a first simulated X-ray measurement based on the current structural estimate of the region of interest.

A simulated X-ray measurement, as referred to herein, represents the expected output of an X-ray detector element when an X-ray source projects one or more X-ray beams through the region of interest towards the X-ray detector element. The simulated X-ray measurement can provide an expected image output that is to be received from the X-ray detector element.

The software programs 144 can receive a first real X-ray measurement from a CBCT system (or other CT imaging system, such as an enclosed gantry helical multi-slice CT with a curved detector or tomotherapy system) and generate an update to the current structural estimate of the region of interest as a function of the first simulated X-ray measurement and the first real X-ray measurement. A real X-ray measurement, as referred to herein, is an actual output that is received from a CBCT system (or other CT imaging system, such as an enclosed gantry helical multi-slice CT with a curved detector or tomotherapy system) that represents the amount of X-rays received by and detected by the X-ray detector along different directions, such as in an image form.

The update can be generated invariant on (independent of) the current structural estimate. The structural estimate can be used to control one or more radiotherapy treatment parameters by recalculating dose, adjusting one or more radiotherapy treatment machine parameters, or generating a display of the structural estimate on a graphical user interface.

In some embodiments, the software programs 144 generate the first simulated X-ray measurement based on the current structural estimate of the region of interest by applying the current structural estimate to a model that generates an expected output of a real X-ray measurement. In some implementations, the model that generates an expected output of a real X-ray measurement includes a modeling function representing beam hardening from a polyenergetic source. In some implementations, the modeling function that generates an expected output of a real X-ray measurement includes a machine learning technique that is trained to establish a relationship between a training real X-ray measurement and a training known simulated X-ray measurement. In some implementations, the modeling function that generates an expected output of a real X-ray measurement includes a linear model or a non-linear model that fits X-ray data to some nominal value comprising at least one of relative electron density, mass density, monoenergetic attenuation, proton stopping power, or bone mineral density.

As an example, the model generates the expected output of the real X-ray measurement, for a given measurement index i per number of X-ray detector elements in the CBCT system, in accordance with Equation 1: ${z}_{i} = {b}_{i} * exp \left(-{\left[Ax\right]}_{i}\right) + {r}_{i} ,$ (1) where b is an incident intensity of flood field, A is a system projection matrix describing a combination of each image pixel at each detector element, *x* is the current X-ray attenuation map, *exp* is an exponential function, and *r* is background noise including scatter. For example, the model applies the current structural estimate to Equation 1 as *x* to output the first simulated measurement *z.* The first simulated measurement can be generated for each of a plurality of X-ray detector elements that are in the CBCT system.

In an example, the value for *r* representing the noise including scatter can be re-estimated based on the current structural estimate at each iteration. For example, the noise including scatter for a given iteration can be applied to another machine leaming technique or model including x (e.g., the current structural estimate) to generate an update and re-estimate the value of the noise including scatter.

In some embodiments, the software programs 144 compute the update to the current structural estimate as a derivative of a statistical objective function with respect to the first simulated X-ray measurement. For example, the statistical objective function can be computed according to Equation 2: ${A}^{T} \left(y./\left(b.*exp\left(-Ax\right)+r\right)-1\right)$ (2) where A is a system projection matrix describing a combination of each image pixel at each detector element, *A^{T}* is a transpose of the system projection matrix, *y is* the first real X-ray measurement, *b* is an incident intensity of flood field, *x* is the current X-ray attenuation map, *exp* is an exponential function, and *r* is background noise including scatter. The update to the current structural estimate represents a perturbation to the current structural estimate. Namely, the update is linearly projected to the current structural estimate into an image space to form a perturbation. The perturbation is scaled by a scalar for stability and the scaled perturbation is subtracted from the current structural estimate to generate an updated structural estimate.

In some implementations, after the structural estimate is updated, at least one of regularization, momentum or denoising is applied to the updated structural estimate to improve the image quality of the 3D model represented by the structural estimate.

In some embodiments, the computation of the update and the updating of the structural estimate is conditioned on and performed in response to a computation of a negative log-likelihood (NLL) function (e.g., a loss function). Specifically, the loss function is computed as a combination of the first simulated X-ray measurement and the first real X-ray measurement, such as in accordance with Equation 3: $NLL = sum \left({z}_{i}-{y}_{i}*log\left({z}_{i}\right)\right)$ (3) which may represent a sum of the differences between each of the plurality of simulated and real measurements of the X-ray CBCT system. Namely, a plurality of simulated measurements each associated with a different X-ray detector can be generated in accordance with Equation 1. The corresponding real X-ray measurements can be received from the X-ray detector, such as from the image acquisition device 132. The differences between each simulated measurement and the corresponding real measurement can be computed and summed. If the sum of these differences is below a threshold (e.g., satisfies a stopping criterion), then the update to the structural estimate is not performed. On the other hand, in response to determining that the loss fails to satisfy the stopping criterion (e.g., if the sum of these differences is above the threshold), the update to the current structural estimate is performed.

In some cases, instead of or in addition to conditioning the performance of the update on the sum of the differences or the loss function defined by Equation 3, the stopping criterion (e.g., the condition for not performing the update) can include a difference between adjacent updates falling below a threshold. For example, the software programs 144 can access a previous update value(s) and can generate a new update value based on the currently generated simulated X-ray measurement. The software programs 144 can compute a difference between these two adjacent or subsequent update values to the structural estimate. The software programs 144 can compare this difference to a stopping threshold and if the difference is below the stopping threshold, the software programs 144 may avoid performing the update to the structural estimate. If the difference is above the stopping threshold, the software programs 144 can perform the update to the structural estimate, such as in accordance with Equation 2.

In some cases, instead of or in addition to conditioning the performance of the update on the sum of the differences or the loss function defined by Equation 3, the stopping criterion (e.g., the condition for not performing the update) can include a number of iterations falling below a maximum iteration value and/or an elapsed time falling below a maximum time limit. For example, software programs 144 can keep a running count of the total number of iterations performed in which at each iteration the update to the structural estimate is performed, such as in accordance with Equation 2. The software programs 144 can also start a timer when the first update is performed. If the total number of iterations is less than a maximum iteration value, then the software programs 144 can perform the update to the structural estimate; and if otherwise, then the update is not performed. As another example, if the total time since the first update was performed is less than the maximum time limit, then the software programs 144 can perform the update to the structural estimate; and if otherwise, then the update is not performed.

**In** some cases, instead of or in addition to conditioning the performance of the update on the sum of the differences or the loss function defined by Equation 3, the stopping criterion (e.g., the condition for not performing the update) can include input requesting termination and display of a result. For example, a user input can be received during the process of performing the iterative image reconstruction. The input may request display of the current structural estimate. **In** response, the software programs 144 stop performing the update and display the current structural estimate. User input can be received to resume the process of performing the iterative image reconstruction. **In** response, the software programs 144 perform the last generated update and continue performing additional updates until another stopping criterion is met or satisfied.

For example, the software programs 144 access an updated structural estimate of the region of interest. The software programs 144 generate a second simulated X-ray measurement based on the updated structural estimate of the region of interest, such as in accordance with Equation 1. The software programs 144 receive a second real X-ray measurement from the CBCT system. The software programs 144 generate a further update to the updated structural estimate of the region of interest as a function of the second simulated X-ray measurement and the second real X-ray measurement, such as in accordance with Equation 2.

In some examples, the software programs 144 reduce a number of iterations and efficiency of generating the updates to the structural estimate by portioning the X-ray measurements into measurement groups and only performing an update if a given real X-ray measurement falls into a group associated with updating the structural estimate. Specifically, the software programs 144 receive a plurality of real X-ray measurements. The X-ray measurements are partitioned into measurement groups, in which a first measurement group of the measurement groups corresponds to a group of X-ray measurements used to update the current X-ray attenuation map, and a second measurement group of the measurement groups corresponds to a group of X-ray measurements that is skipped from updating the current X-ray structural estimate. The software programs 144 determines that the first real X-ray measurement falls within the first measurement group and, in response, the software programs 144 perform the update to the current structural estimate.

**In** some cases, the measurement groups are divided by number or quantity of real X-ray measurements that are received. For example, a queue may be maintained in which the real X-ray measurements are input. The queue may include a number of entries. As each new real X-ray measurement is received, the X-ray measurement is input to the queue. When the number of entries in the queue reaches a specified threshold, the last received X-ray measurement or a subset of last received X-ray measurements are used to perform an update to the structural estimate, such as in accordance with Equation 2. For example, when the number of entries in the queue reaches the specified threshold, the software programs 144 use Equation 1 to generate a simulated set of X-ray measurements which are used in combination with the last received X-ray measurement or a subset of last received X-ray measurements to generate the update to the current structural estimate. After the current structural estimate is updated, the queue is flushed, and the entries are deleted so that 0 entries remain in the queue. **In** this way, the next update is performed when the next set of real X-ray measurements fill up the entries in the queue to reach the specified threshold.

In some implementations, the current structural estimate is smoothed based on a collection of updates to the structural estimate. Specifically, the software programs 144 can add each computed new updated of the structural estimate to a collection of updates performed on the structural estimate. Each update in the collection corresponds to or is associated with a respective iteration of performing updates to the structural estimate. The software programs 144 can identify a pattern in the collected plurality of updates. The software programs 144 can adjust or perform a new update to the structural estimate based on the identified pattern in the collected plurality of updates. For example, the software programs 144 can generate a plurality of updates based on a plurality of previously generated simulated X-ray measurements and corresponding real X-ray measurements. Each of these updates is input to a collection and a machine learning model or heuristic is applied to the collection to identify a particular pattern. The software programs 144 can then generate a new simulated X-ray measurement and receive a new real X-ray measurement. The software programs 144 can compute a new update to the structural estimate based on the new simulated X-ray measurement and new real X-ray measurement, according to Equation 2. The software programs 144 can adjust the computed new update based on the particular pattern that has been identified in the collection of previous updates. This can smooth the updates by preventing performance of updates that are too large in a particular portion of the structural estimate.

In addition to the memory device 116 storing the software programs 144, it is contemplated that software programs 144 may be stored on a removable computer medium, such as a hard drive, a computer disk, a CD-ROM, a DVD, a HD, a Blu-Ray DVD, USB flash drive, a SD card, a memory stick, or any other suitable medium; and the software programs 144 when downloaded to image processing device 112 may be executed by image processor 114.

The processor 114 may be communicatively coupled to the memory device 116, and the processor 114 may be configured to execute computer-executable instructions stored thereon. The processor 114 may send or receive medical images 146 to memory device 116. For example, the processor 114 may receive medical images 146 from the image acquisition device 132 via the communication interface 118 and network 120 to be stored in memory device 116. The processor 114 may also send medical images 146 stored in memory device 116 via the communication interface 118 to the network 120 be either stored in database 124 or the hospital database 126.

Further, the processor 114 may utilize software programs 144 (e.g., a treatment planning software) along with the medical images 146 and patient data 145 to create the radiation therapy treatment plan 142. Medical images 146 may include information such as imaging data associated with a patient anatomical region, organ, or volume of interest segmentation data. Patient data 145 may include information such as (1) functional organ modeling data (e.g., serial versus parallel organs, appropriate dose response models, etc.); (2) radiation dosage data (e.g., DVH information); or (3) other clinical information about the patient and course of treatment (e.g., other surgeries, chemotherapy, previous radiotherapy, etc.).

In addition, the processor 114 may utilize software programs to generate intermediate data such as updated parameters to be used, for example, by a machine learning model, such as a neural network model; or generate intermediate 2D or 3D images, which may then subsequently be stored in memory device 116. The processor 114 may subsequently transmit the executable radiation therapy treatment plan 142 via the communication interface 118 to the network 120 to the radiation therapy device 130, where the radiation therapy plan will be used to treat a patient with radiation. In addition, the processor 114 may execute software programs 144 to implement functions such as image conversion, image segmentation, deep learning, neural networks, and artificial intelligence. For instance, the processor 114 may execute software programs 144 that train or contour a medical image; such software programs144 when executed may train a boundary detector or utilize a shape dictionary.

The processor 114 may be a processing device, including one or more general-purpose processing devices such as a microprocessor, a central processing unit (CPU), a graphics processing unit (GPU), an accelerated processing unit (APU), or the like. More particularly, the processor 114 may be a complex instruction set computing (CISC) microprocessor, a reduced instruction set computing (RISC) microprocessor, a very long instruction Word (VLIW) microprocessor, a processor implementing other instruction sets, or processors implementing a combination of instruction sets. The processor 114 may also be implemented by one or more special-purpose processing devices such as an application-specific integrated circuit (ASIC), a field programmable gate array (FPGA), a digital signal processor (DSP), a System on a Chip (SoC), or the like. As would be appreciated by those skilled in the art, in some embodiments, the processor 114 may be a special-purpose processor rather than a general-purpose processor. The processor 114 may include one or more known processing devices, such as a microprocessor from the Pentium^{™}, Core^{™}, Xeon^{™}, or Itanium^{®} family manufactured by Intel^{™}, the Turion^{™}, Athlon^{™}, Sempron^{™}, Opteron^{™}, FX^{™}, Phenom^{™} family manufactured by AMD^{™}, or any of various processors manufactured by Sun Microsystems. The processor 114 may also include graphical processing units such as a GPU from the GeForce^{®}, Quadro^{®}, Tesla^{®} family manufactured by Nvidia^{™}, GMA, Iris^{™} family manufactured by Intel^{™}, or the Radeon^{™} family manufactured by AMD^{™}. The processor 114 may also include accelerated processing units such as the Xeon Phi^{™} family manufactured by Intel^{™}. The disclosed embodiments are not limited to any type of processor(s) otherwise configured to meet the computing demands of identifying, analyzing, maintaining, generating, and/or providing large amounts of data or manipulating such data to perform the methods disclosed herein. In addition, the term "processor" may include more than one processor (for example, a multi-core design or a plurality of processors each having a multi-core design). The processor 114 can execute sequences of computer program instructions, stored in memory device 116, to perform various operations, processes, methods that will be explained in greater detail below.

The memory device 116 can store medical images 146. In some embodiments, the medical images 146 may include one or more MRI images (e.g., 2D MRI, 3D MRI, 2D streaming MRI, four-dimensional (4D) MRI, 4D volumetric MRI, 4D cine MRI, etc.), functional MRI images (e.g., fMRI, DCE-MRI, diffusion MRI), CT images (e.g., 2D CT, cone beam CT, 3D CT, 4D CT), ultrasound images (e.g., 2D ultrasound, 3D ultrasound, 4D ultrasound), one or more projection images representing views of an anatomy depicted in the MRI, synthetic CT (pseudo-CT), and/or CT images at different angles of a gantry relative to a patient axis, PET images, X-ray images, fluoroscopic images, radiotherapy portal images, SPECT images, computer generated synthetic images (e.g., pseudo-CT images), aperture images, graphical aperture image representations of MLC leaf positions at different gantry angles, and the like. Further, the medical images 146 may also include medical image data, for instance, training images, ground truth images, contoured images, and dose images. In an embodiment, the medical images 146 may be received from the image acquisition device 132. Accordingly, the image acquisition device 132 may include an MRI imaging device, an MV imaging device, a CT imaging device, a CBCT imaging device, a PET imaging device, an ultrasound imaging device, a fluoroscopic device, a SPECT imaging device, an integrated linac and MRI imaging device, an integrated linac and CT imaging device, an integrated linac and CBCT imaging device, or other medical imaging devices for obtaining the medical images of the patient. The medical images 146 may be received and stored in any type of data or any type of format that the image processing device 112 may use to perform operations consistent with the disclosed embodiments.

The memory device 116 may be a non-transitory computer-readable medium, such as a read-only memory (ROM), a phase-change random access memory (PRAM), a static random access memory (SRAM), a flash memory, a random access memory (RAM), a dynamic random access memory (DRAM) such as synchronous DRAM (SDRAM), an electrically erasable programmable read-only memory (EEPROM), a static memory (e.g., flash memory, flash disk, static random access memory) as well as other types of random access memories, a cache, a register, a CD-ROM, a DVD or other optical storage, a cassette tape, other magnetic storage device, or any other non-transitory medium that may be used to store information including image, data, or computer-executable instructions (e.g., stored in any format) capable of being accessed by the processor 114, or any other type of computer device. The computer program instructions can be accessed by the processor 114, read from the ROM, or any other suitable memory location, and loaded into the RAM for execution by the processor 114. For example, the memory device 116 may store one or more software applications. Software applications stored in the memory device 116 may include, for example, an operating system 143 for common computer systems as well as for software-controlled devices. Further, the memory device 116 may store an entire software application, or only a part of a software application, that is executable by the processor 114. For example, the memory device 116 may store one or more radiation therapy treatment plans 142.

The image processing device 112 can communicate with the network 120 via the communication interface 118, which can be communicatively coupled to the processor 114 and the memory device 116. The communication interface 118 may provide communication connections between the image processing device 112 and radiotherapy system 100 components (e.g., permitting the exchange of data with external devices). For instance, the communication interface 118 may, in some embodiments, have appropriate interfacing circuitry to connect to the user interface 136, which may be a hardware keyboard, a keypad, or a touch screen through which a user may input information into radiotherapy system 100.

Communication interface 118 may include, for example, a network adaptor, a cable connector, a serial connector, a USB connector, a parallel connector, a high-speed data transmission adaptor (e.g., such as fiber, USB 3.0, thunderbolt, and the like), a wireless network adaptor (e.g., such as a Wi-Fi adaptor), a telecommunication adaptor (e.g., 3G, 4G/LTE and the like), and the like. Communication interface 118 may include one or more digital and/or analog communication devices that permit image processing device 112 to communicate with other machines and devices, such as remotely located components, via the network 120.

The network 120 may provide the functionality of a local area network (LAN), a wireless network, a cloud computing environment (e.g., software as a service, platform as a service, infrastructure as a service, etc.), a client-server, a wide area network (WAN), and the like. For example, network 120 may be a LAN or a WAN that may include other systems S1 (138), S2 (140), and S3 (141). Systems S1, S2, and S3 may be identical to image processing device 112 or may be different systems. In some embodiments, one or more systems in network 120 may form a distributed computing/simulation environment that collaboratively performs the embodiments described herein. In some embodiments, one or more systems S1, S2, and S3 may include a CT scanner that obtains CT images (e.g., medical images 146). In addition, network 120 may be connected to Internet 122 to communicate with servers and clients that reside remotely on the internet.

Therefore, network 120 can allow data transmission between the image processing device 112 and a number of various other systems and devices, such as the OIS 128, the radiation therapy device 130, and the image acquisition device 132. Further, data generated by the OIS 128 and/or the image acquisition device 132 may be stored in the memory device 116, the database 124, and/or the hospital database 126. The data may be transmitted/received via network 120, through communication interface 118 in order to be accessed by the processor 114, as required.

The image processing device 112 may communicate with database 124 through network 120 to send/receive a plurality of various types of data stored on database 124. For example, database 124 may include machine data (control points) that includes information associated with a radiation therapy device 130, image acquisition device 132, or other machines relevant to radiotherapy. Machine data information may include control points, such as radiation beam size, arc placement, beam on and off time duration, machine parameters, segments, MLC configuration, gantry speed, MRI pulse sequence, and the like. Database 124 may be a storage device and may be equipped with appropriate database administration software programs. One skilled in the art would appreciate that database 124 may include a plurality of devices located either in a central or a distributed manner.

In some embodiments, database 124 may include a processor-readable storage medium (not shown). While the processor-readable storage medium in an embodiment may be a single medium, the term "processor-readable storage medium" should be taken to include a single medium or multiple media (e.g., a centralized or distributed database, and/or associated caches and servers) that store the one or more sets of computer-executable instructions or data. The term "processor-readable storage medium" shall also be taken to include any medium that is capable of storing or encoding a set of instructions for execution by a processor and that cause the processor to perform any one or more of the methodologies of the present disclosure. The term "processor-readable storage medium" shall accordingly be taken to include, but not be limited to, solid-state memories and optical and magnetic media. For example, the processor-readable storage medium can be one or more volatile, non-transitory, or non-volatile tangible computer-readable media.

Image processor 114 may communicate with database 124 to read images into memory device 116 or store images from memory device 116 to database 124. For example, the database 124 may be configured to store a plurality of images (e.g., 3D MRI, 4D MRI, 2D MRI slice images, CT images, 2D Fluoroscopy images, X-ray images, raw data from MR scans or CT scans, Digital Imaging and Communications in Medicine (DIMCOM) data, projection images, graphical aperture images, etc.) that the database 124 received from image acquisition device 132. Database 124 may store data to be used by the image processor 114 when executing software program 144 or when creating radiation therapy treatment plans 142. Database 124 may store the data produced by the trained machine leaming mode, such as a neural network including the network parameters constituting the model learned by the network and the resulting estimated data. As referred to herein, "estimate" or "estimated" can be used interchangeably with "predict" or "predicted" and should be understood to have the same meaning. The image processing device 112 may receive the imaging data, such as a medical image 146 (e.g., 2D MRI slice images, CT images, 2D Fluoroscopy images, X-ray images, 3DMRI images, 4D MRI images, projection images, graphical aperture images, image contours, etc.) from the database 124, the radiation therapy device 130 (e.g., an MR-linac), and/or the image acquisition device 132 to generate a treatment plan 142.

In an embodiment, the radiotherapy system 100 can include an image acquisition device 132 that can acquire medical images (e.g., MRI images, 3D MRI, 2D streaming MRI, 4D volumetric MRI, CT images, cone-Beam CT, PET images, functional MRI images (e.g., fMRI, DCE-MRI, and diffusion MRI), X-ray images, fluoroscopic image, ultrasound images, radiotherapy portal images, SPECT images, and the like) of the patient. Image acquisition device 132 may, for example, be an MRI imaging device, a CT imaging device, a PET imaging device, an ultrasound device, a fluoroscopic device, a SPECT imaging device, or any other suitable medical imaging device for obtaining one or more medical images of the patient. Images acquired by the image acquisition device 132 can be stored within database 124 as either imaging data and/or test data. By way of example, the images acquired by the image acquisition device 132 can be also stored by the image processing device 112 as medical images 146 in memory device 116.

In an embodiment, for example, the image acquisition device 132 may be integrated with the radiation therapy device 130 as a single apparatus (e.g., an MR-linac). Such an MR-linac can be used, for example, to determine a location of a target organ or a target tumor in the patient, so as to direct radiation therapy accurately according to the radiation therapy treatment plan 142 to a predetermined target.

The image acquisition device 132 can be configured to acquire one or more images of the patient's anatomy for a region of interest (e.g., a target organ, a target tumor, or both). Each image, typically a 2D image or slice, can include one or more parameters (e.g., a 2D slice thickness, an orientation, and a location, etc.). In an embodiment, the image acquisition device 132 can acquire a 2D slice in any orientation. For example, an orientation of the 2D slice can include a sagittal orientation, a coronal orientation, or an axial orientation. The processor 114 can adjust one or more parameters, such as the thickness and/or orientation of the 2D slice, to include the target organ and/or target tumor. In an embodiment, 2D slices can be determined from information such as a 3D MRI volume. Such 2D slices can be acquired by the image acquisition device 132 in "real-time" while a patient is undergoing radiation therapy treatment, for example, when using the radiation therapy device 130, with "real-time" meaning acquiring the data in at least milliseconds or less.

The image processing device 112 may generate and store radiation therapy treatment plans 142 for one or more patients. The radiation therapy treatment plans 142 may provide information about a particular radiation dose to be applied to each patient. The radiation therapy treatment plans 142 may also include other radiotherapy information, such as control points including beam angles, gantry angles, beam intensity, dose-histogram-volume information, the number of radiation beams to be used during therapy, the dose per beam, and the like.

The image processor 114 may generate the radiation therapy treatment plan 142 by using software programs 144 such as treatment planning software (such as Monaco^{®}, manufactured by Elekta AB of Stockholm, Sweden). In order to generate the radiation therapy treatment plans 142, the image processor 114 may communicate with the image acquisition device 132 (e.g., a CT device, an MRI device, a PET device, an X-ray device, an ultrasound device, etc.) to access images of the patient and to delineate a target, such as a tumor, to generate contours of the images. In some embodiments, the delineation of one or more OARs, such as healthy tissue surrounding the tumor or in close proximity to the tumor, may be required. Therefore, segmentation of the OAR may be performed when the OAR is close to the target tumor. In addition, if the target tumor is close to the OAR (e.g., prostate in near proximity to the bladder and rectum), then by segmenting the OAR from the tumor, the radiotherapy system 100 may study the dose distribution not only in the target but also in the OAR.

In order to delineate a target organ or a target tumor from the OAR, medical images, such as MRI images, CT images, PET images, fMRI images, X-ray images, ultrasound images, radiotherapy portal images, SPECT images, and the like, of the patient undergoing radiotherapy may be obtained non-invasively by the image acquisition device 132 to reveal the internal structure of a body part. Based on the information from the medical images, a 3D structure of the relevant anatomical portion may be obtained and used to generate a contour of the image. Contours of the image can include data overlaid on top of the image that delineates one or more structures of the anatomy. In some cases, the contours can be files associated with respective images that specify the coordinates or 2D or 3D locations of various structures of the anatomy depicted in the images.

In addition, during a treatment planning process, many parameters may be taken into consideration to achieve a balance between efficient treatment of the target tumor (e.g., such that the target tumor receives enough radiation dose for an effective therapy) and low irradiation of the OAR(s) (e.g., the OAR(s) receives as low a radiation dose as possible). Other parameters that may be considered include the location of the target organ and the target tumor, the location of the OAR, and the movement of the target in relation to the OAR. For example, the 3D structure may be obtained by contouring the target or contouring the OAR within each 2D layer or slice of an **MRI** or CT image and combining the contour of each 2D layer or slice. The contour may be generated manually (e.g., by a physician, dosimetrist, or health care worker using a program such as MONACO^{™} manufactured by Elekta AB of Stockholm, Sweden) or automatically (e.g., using a program such as the Atlas-based auto-segmentation software, ABAS^{™}, manufactured by Elekta AB of Stockholm, Sweden). In certain embodiments, the 3D structure of a target tumor or an OAR may be generated automatically by the treatment planning software.

After the target tumor and the OAR(s) have been located and delineated, a dosimetrist, physician, or healthcare worker may determine a dose of radiation to be applied to the target tumor, as well as any maximum amounts of dose that may be received by the OAR proximate to the tumor (e.g., left and right parotid, optic nerves, eyes, lens, inner ears, spinal cord, brain stem, and the like). After the radiation dose is determined for each anatomical structure (e.g., target tumor, OAR), a process known as inverse planning may be performed to determine one or more treatment plan parameters that would achieve the desired radiation dose distribution. Examples of treatment plan parameters include volume delineation parameters (e.g., which define target volumes, contour sensitive structures, etc.), margins around the target tumor and OARs, beam angle selection, collimator settings, and beam-on times. During the inverse-planning process, the physician may define dose constraint parameters that set bounds on how much radiation an OAR may receive (e.g., defining full dose to the tumor target and zero dose to any OAR; defining 95% of dose to the target tumor; defining that the spinal cord, brain stem, and optic structures receive ≤ 45Gy, ≤ 55Gy and < 54Gy, respectively). The result of inverse planning may constitute a radiation therapy treatment plan 142 that may be stored in memory device 116 or database 124. Some of these treatment parameters may be correlated. For example, tuning one parameter (e.g., weights for different objectives, such as increasing the dose to the target tumor) in an attempt to change the treatment plan may affect at least one other parameter, which in turn may result in the development of a different treatment plan. Thus, the image processing device 112 can generate a tailored radiation therapy treatment plan 142 having these parameters in order for the radiation therapy device 130 to provide radiotherapy treatment to the patient.

In addition, the radiotherapy system 100 may include a display device 134 and a user interface 136. The display device 134 may include one or more display screens that display medical images, interface information, treatment planning parameters (e.g., projection images, graphical aperture images, contours, dosages, beam angles, etc.) treatment plans, a target, localizing a target and/or tracking a target, or any related information to the user. The user interface 136 may be a keyboard, a keypad, a touch screen, or any type of device that a user may use to input information to radiotherapy system 100. Alternatively, the display device 134 and the user interface 136 may be integrated into a device such as a tablet computer (e.g., Apple iPad^{®}, Lenovo Thinkpad^{®}, Samsung Galaxy ^{®}, etc.).

Furthermore, any and all components of the radiotherapy system 100 may be implemented as a virtual machine (e.g., VMWare, Hyper-V, and the like). For instance, a virtual machine can be software that functions as hardware. Therefore, a virtual machine can include at least one or more virtual processors, one or more virtual memories, and one or more virtual communication interfaces that together function as hardware. For example, the image processing device 112, the OIS 128, and the image acquisition device 132 could be implemented as a virtual machine. Given the processing power, memory, and computational capability available, the entire radiotherapy system 100 could be implemented as a virtual machine.

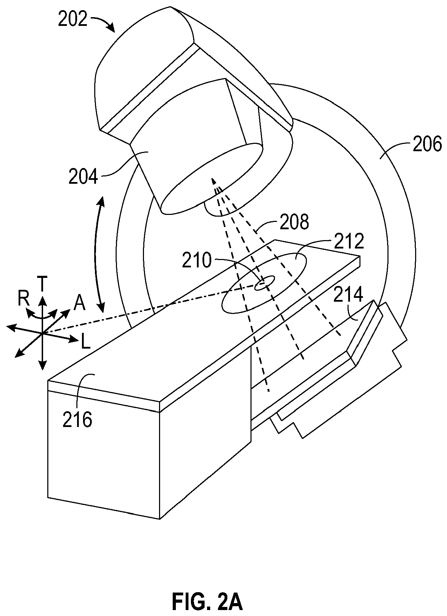

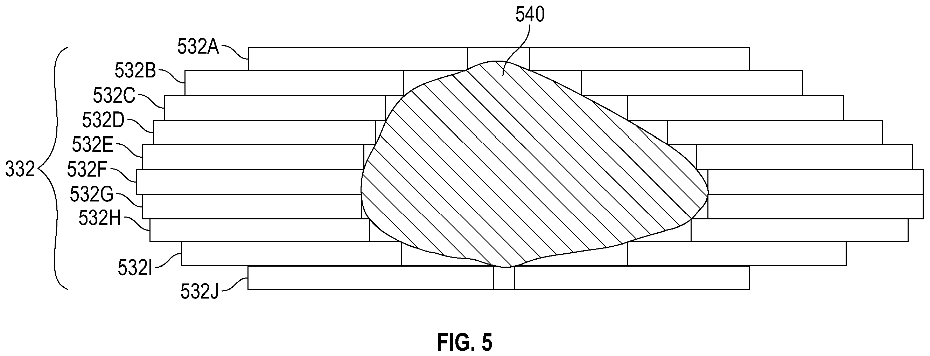

FIG. 2A illustrates an example of a radiation therapy device 202 that may include a radiation source, such as an X-ray source or a linear accelerator, a couch 216, an imaging detector 214, and a radiation therapy output 204, such as an MV source. The radiation therapy device 202 may be configured to emit a radiation beam 208, such as an MV treatment beam, to provide therapy to a patient. The radiation therapy output 204 can include one or more attenuators or collimators, such as an MLC as described in the illustrative embodiment of FIG. 5, below.

Referring back to FIG. 2A, a patient can be positioned in a region 212 and supported by the treatment couch 216 to receive a radiation therapy dose, according to a radiation therapy treatment plan. The radiation therapy output 204 can be mounted or attached to a gantry 206 or other mechanical support. One or more chassis motors (not shown) may rotate the gantry 206 and the radiation therapy output 204 around couch 216 when the couch 216 is inserted into the treatment area. In an embodiment, gantry 206 may be continuously rotatable around couch 216 when the couch 216 is inserted into the treatment area. In another embodiment, gantry 206 may rotate to a predetermined position when the couch 216 is inserted into the treatment area. For example, the gantry 206 can be configured to rotate the therapy output 204 around an axis ("A"). Both the couch 216 and the radiation therapy output 204 can be independently moveable to other positions around the patient, such as moveable in transverse direction ("*T*"), moveable in a lateral direction ("*L*"), or as rotation about one or more other axes, such as rotation about a transverse axis (indicated as *"R"*)*.* A controller communicatively connected to one or more actuators (not shown) may control the couch's 216 movements or rotations in order to properly position the patient in or out of the radiation beam 208 according to a radiation therapy treatment plan. Both the couch 216 and the gantry 206 are independently moveable from one another in multiple degrees of freedom, which allows the patient to be positioned such that the radiation beam 208 can precisely target the tumor. The MLC may be integrated and included within gantry 206 to deliver the radiation beam 208 of a certain shape.

The coordinate system (including axes *A, T,* and *L*) shown in FIG. 2A can have an origin located at an isocenter 210. The isocenter 210 can be defined as a location where the central axis of the radiation beam 208 intersects the origin of a coordinate axis, such as to deliver a prescribed radiation dose to a location on or within a patient. Alternatively, the isocenter 210 can be defined as a location where the central axis of the radiation beam 208 intersects the patient for various rotational positions of the radiation therapy output 204 as positioned by the gantry 206 around the axis *A.* As discussed herein, the gantry angle corresponds to the position of gantry 206 relative to axis *A,* although any other axis or combination of axes can be referenced and used to determine the gantry angle.

Gantry 206 may also have an attached imaging detector 214. The imaging detector 214 is preferably located opposite to the radiation source, and in an embodiment, the imaging detector 214 can be located within a field of the therapy beam 208. The imaging detector 214 can capture an image from the radiation emitted by the radiation therapy output 204, such as an MV X-ray source, and as attenuated by the patient. In this manner, the MV imaging panel can capture an MV image using the treatment beam itself. The MV image can provide information about the internal structure of the patient.

As an example, the imaging detector 214 can be referred to as an electronic portal imaging device (EPID). In some examples, an EPID can include a phosphor-based flat panel detector. A metal plate can be used as a first layer to increase signal. X-ray photons interact with the metal plate to produce high energy electrons, which subsequently deposit energy along their path in a phosphor layer. This deposited energy leads to the creation of optical photons, which transport within the phosphor and can be detected, such as by an array of thin film transistors (TFTs). These TFTs can be read out, such as to generate 2D images. Other types of EPIDs can also be used.

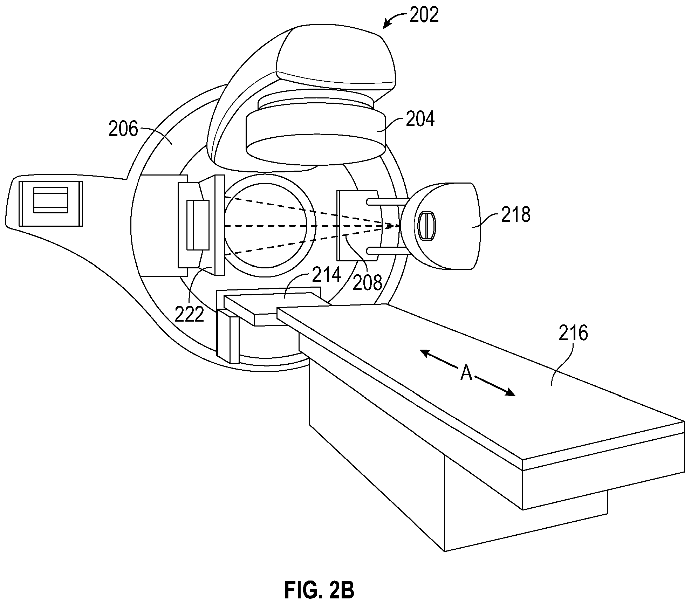

Some advantages of using MV imaging can include a) images directly capture the treatment beam's eye view, and thus include the most important components of motion; and b) there is no additional imaging dose to the patient. However, these MV images can suffer from low image quality, primarily due to one or more of the following:
a) low tissue contrast at MV energies;
b) degradation due to scatter;
c) low absorption probability of X-ray photons within the detector (a large fraction go through without interacting), which increases quantum noise;
d) scatter of photons and electrons within the detector itself, which degrades spatial resolution; and
e) finite focal spot size of treatment beam.
Furthermore, the MLC can move dynamically during treatment in order to sculpt the treatment dose, capturing only a small segment of the patient's anatomy at any given time, which may not even intersect with the target. As a result, many radiotherapy systems also include a diagnostic kV source for imaging, as described below with respect to FIG. 2B.

The imaging detector 214 can be mounted on the gantry 206 (preferably opposite the radiation therapy output 204), such as to maintain alignment with the therapy beam 208. The imaging detector 214 rotates about the rotational axis as the gantry 206 rotates. In an embodiment, the imaging detector 214 can be a flat panel detector (e.g., a direct detector or a scintillator detector). In this manner, the imaging detector 214 can be used to monitor the therapy beam 208 or the imaging detector 214 can be used for imaging the patient's anatomy, such as portal imaging (e.g., to provide real X-ray measurements). The control circuitry of radiation therapy device 202 may be integrated within system 100 or remote from it.

In an illustrative embodiment, one or more of the couch 216, the therapy output 204, or the gantry 206 can be automatically positioned, and the therapy output 204 can establish the therapy beam 208 according to a specified dose for a particular therapy delivery instance. A sequence of therapy deliveries can be specified according to a radiation therapy treatment plan, such as using one or more different orientations or locations of the gantry 206, couch 216, or therapy output 204. The therapy deliveries can occur sequentially, but can intersect in a desired therapy locus on or within the patient, such as at the isocenter 210. A prescribed cumulative dose of radiation therapy can thereby be delivered to the therapy locus while damage to tissue near the therapy locus can be reduced or avoided.

FIG. 2B illustrates an example of a radiation therapy device 202 that may include a combined linac and an imaging system, such as can include a CT imaging system. The radiation therapy device 202 can include an MLC (not shown). The CT imaging system can include an imaging X-ray source 218, such as providing X-ray energy in a kiloelectron-Volt (keV) energy range which can be used for imaging the patient's anatomy, such as portal imaging (e.g., to provide real X-ray measurements). The imaging X-ray source 218 (also referred to as a "kV source") can provide a fan-shaped and/or a conical beam 208 directed to an imaging detector 222, such as a flat panel detector. The radiation therapy device 202 can be similar to the system described in relation to FIG. 2A, such as including a radiation therapy output 204, a gantry 206, a couch 216, and another imaging detector 214 (such as a flat panel detector). The X-ray source 218 can provide a comparatively-lower-energy X-ray diagnostic beam, for imaging.

In the illustrative embodiment of FIG. 2B, the radiation therapy output 204, e.g., MV source, and the X-ray source 218, e.g., kV source, can be mounted on the same rotating gantry 206, rotationally separated from each other by 90 degrees. This arrangement can enable imaging perpendicular to the beam of radiation output by radiation therapy output 204, which, in some embodiments, can be a Megavolt (MV) treatment beam. The kV source 218 can be used to acquire 2D X-ray projections as the kV source 218 moves around the patient along gantry 206.

In another embodiment, two or more X-ray sources can be mounted along the circumference of the gantry 206, such as each having its own detector arrangement to provide multiple angles of diagnostic imaging concurrently. Similarly, multiple radiation therapy outputs 204 can be provided.

"Patient representation" or "patient state" is some virtual description of the patient's 3D anatomy at a given time. For example, patient representation can include a 3D reconstructed image of the patient. The patient representation can include one or more structures extracted from a 3D image. The patient representation can be a 3D deformation vector field (DVF), which allows one to deform a previous reference image to be representative of the current time. In some examples, the patient representation can include a biomechanical model.

In the present approach, and as described in detail below, as the 3D patient representation evolves during the treatment, an MV image (or CT image) can be simulated as accurately as possible, taking all of the imaging processes into account so that the simulated MV images (or simulated CT images) can provide a near-exact match to the actual MV images. The 2D simulated MV images (or 2D simulated CT images) can be compared to actual MV images (or CT images) acquired throughout the treatment. Significant differences observed between the actual and simulated images can provide an indication that the 3D patient representation is no longer representative of the actual patient anatomy. In such a case, the treatment can be interrupted, and a new 3D patient representation can be generated before re-commencing the treatment. For example, a new 3D image can be acquired, or a new respiratory model can be generated that relates partial patient data to 3D image representations.

In this manner, a radiotherapy system can implement a quality assurance technique that can be used in parallel with the radiotherapy treatment. By using a 3D patient representation that is being updated in real-time, various techniques of this disclosure can simulate what the MV image (or CT image) should look like and the simulated MV image (or simulated CT image) can be compared to an actual MV image (or actual CT image). To do this, various techniques of this disclosure can determine how the treatment beam interacts with the patient and the detector.

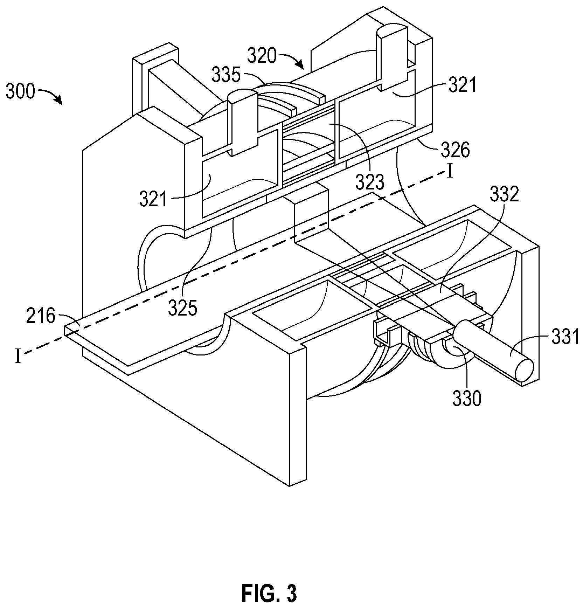

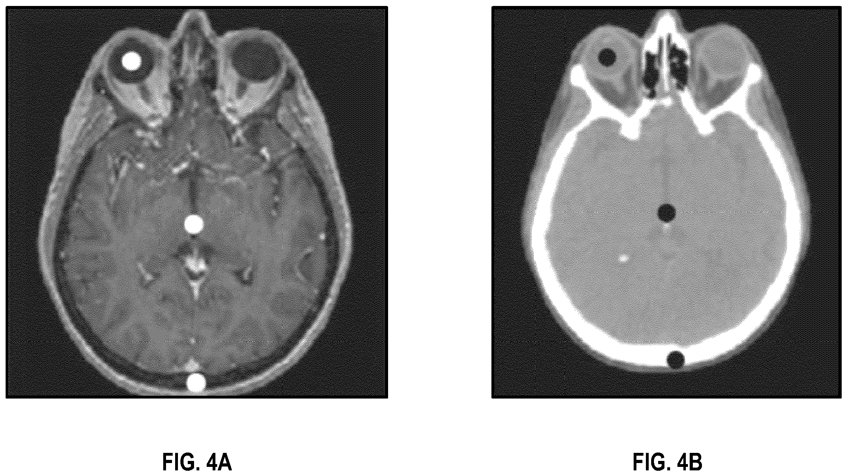

FIG. 3 depicts an example radiation therapy system 300 that can include combining a radiation therapy device 202 and an imaging system, such as a nuclear MR imaging system (e.g., known in the art as an MR-linac) consistent with the disclosed embodiments. As shown, system 300 may include a couch 216, an image acquisition device 320, and a radiation delivery device 330. System 300 delivers radiation therapy to a patient in accordance with a radiotherapy treatment plan. In some embodiments, image acquisition device 320 may correspond to image acquisition device 132 in FIG. 1 that may acquire origin images of a first modality (e.g., MRI image shown in FIG. 4A) or destination images of a second modality (e.g., CT image shown in FIG. 4B).

Couch 216 may support a patient (not shown) during a treatment session. In some implementations, couch 216 may move along a horizontal translation axis (labelled "I"), such that couch 216 can move the patient resting on couch 216 into and/or out of system 300. Couch 216 may also rotate around a central vertical axis of rotation, transverse to the translation axis. To allow such movement or rotation, couch 216 may have motors (not shown) enabling the couch 216 to move in various directions and to rotate along various axes. A controller (not shown) may control these movements or rotations in order to properly position the patient according to a treatment plan.

In some embodiments, image acquisition device 320 may include an MRI machine used to acquire 2D or 3D MRI images of the patient before, during, and/or after a treatment session. Image acquisition device 320 may include a magnet 321 for generating a primary magnetic field for magnetic resonance imaging. The magnetic field lines generated by operation of magnet 321 may run substantially parallel to the central translation axis I. Magnet 321 may include one or more coils with an axis that runs parallel to the translation axis I. In some embodiments, the one or more coils in magnet 321 may be spaced such that a central window 323 of magnet 321 is free of coils. **In** other embodiments, the coils in magnet 321 may be thin enough or of a reduced density such that they are substantially transparent to radiation of the wavelength generated by radiotherapy device 330. Image acquisition device 320 may also include one or more shielding coils, which may generate a magnetic field outside magnet 321 of approximately equal magnitude and opposite polarity in order to cancel or reduce any magnetic field outside of magnet 321. As described below, radiation source 331 of radiotherapy device 330 may be positioned in the region where the magnetic field is cancelled, at least to a first order, or reduced.

Image acquisition device 320 may also include two gradient coils 325 and 326, which may generate a gradient magnetic field that is superposed on the primary magnetic field. Coils 325 and 326 may generate a gradient in the resultant magnetic field that allows spatial encoding of the protons so that their position can be determined. Gradient coils 325 and 326 may be positioned around a common central axis with the magnet 321 and may be displaced along that central axis. The displacement may create a gap, or window, between coils 325 and 326. In embodiments where magnet 321 can also include a central window 323 between coils, the two windows may be aligned with each other.

In some embodiments, image acquisition device 320 may be an imaging device other than an MRI, such as an X-ray, a CT, a CBCT, a spiral CT, a PET, a SPECT, an optical tomography, a fluorescence imaging, ultrasound imaging, radiotherapy portal imaging device, or the like. As would be recognized by one of ordinary skill in the art, the above description of image acquisition device 320 concerns certain embodiments and is not intended to be limiting.

Radiotherapy device 330 may include the radiation source 331, such as an X-ray source or a linac, and an MLC 332 (shown below in FIG. 5). Radiotherapy device 330 may be mounted on a chassis 335. One or more chassis motors (not shown) may rotate chassis 335 around couch 216 when couch 216 is inserted into the treatment area. In an embodiment, chassis 335 may be continuously rotatable around couch 216 when couch 216 is inserted into the treatment area. Chassis 335 may also have an attached radiation detector (not shown), preferably located opposite to radiation source 331 and with the rotational axis of chassis 335 positioned between radiation source 331 and the detector. Further, device 330 may include control circuitry (not shown) used to control, for example, one or more of couch 216, image acquisition device 320, and radiotherapy device 330. The control circuitry of radiotherapy device 330 may be integrated within system 300 or remote from it.

During a radiotherapy treatment session, a patient may be positioned on couch 216. System 300 may then move couch 216 into the treatment area defined by magnet 321, coils 325 and 326, and chassis 335. Control circuitry may then control radiation source 331, MLC 332, and the chassis motor(s) to deliver radiation to the patient through the window between coils 325 and 326 according to a radiotherapy treatment plan.

FIG. 2A, FIG. 2B, and FIG. 3 illustrate generally embodiments of a radiation therapy device configured to provide radiotherapy treatment to a patient, including a configuration where a radiation therapy output can be rotated around a central axis (e.g., an axis "A"). Other radiation therapy output configurations can be used. For example, a radiation therapy output can be mounted to a robotic arm or manipulator having multiple degrees of freedom. In yet another embodiment, the therapy output can be fixed, such as located in a region laterally separated from the patient, and a platform supporting the patient can be used to align a radiation therapy isocenter with a specified target locus within the patient.

As discussed above, radiation therapy devices described by FIG. 2A, FIG. 2B, and FIG. 3 include an MLC for shaping, directing, or modulating an intensity of a radiation therapy beam to the specified target locus within the patient.

FIG. 5 illustrates an example of an MLC 332 that includes leaves 532A through 532J that can be automatically positioned to define an aperture approximating a tumor 540 cross section or projection. The leaves 532A through 532J permit modulation of the radiation therapy beam. The leaves 532A through 532J can be made of a material specified to attenuate or block the radiation beam in regions other than the aperture, in accordance with the radiation treatment plan. For example, the leaves 532A through 532J can include metallic plates, such as comprising tungsten, with a long axis of the plates oriented parallel to a beam direction and having ends oriented orthogonally to the beam direction (as shown in the plane of the illustration of FIG. 2A). A "state" of the MLC 332 can be adjusted adaptively during a course of radiation therapy treatment, such as to establish a therapy beam that better approximates a shape or location of the tumor 540 or another target locus. This is in comparison to using a static collimator configuration or as compared to using an MLC 332 configuration determined exclusively using an "offline" therapy planning technique. A radiation therapy technique using the MLC 332 to produce a specified radiation dose distribution to a tumor or to specific areas within a tumor can be referred to as IMRT.

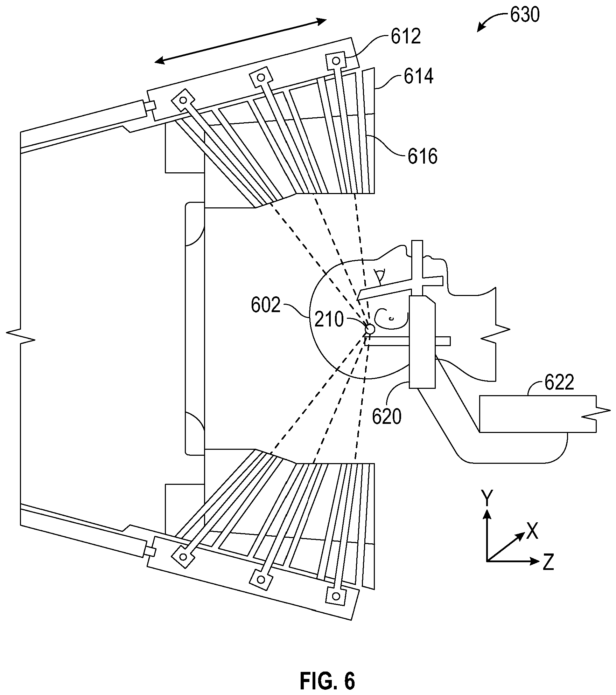

FIG. 6 illustrates an embodiment of another type of radiotherapy device 630 (e.g., a Leksell Gamma Knife), according to some embodiments of the present disclosure. As shown in FIG. 6, in a radiotherapy treatment session, a patient 602 may wear a coordinate frame 620 to keep stable the patient's body part (e.g., the head) undergoing surgery or radiotherapy. Coordinate frame 620 and a patient positioning system 622 may establish a spatial coordinate system, which may be used while imaging a patient or during radiation surgery. Radiotherapy device 630 may include a protective housing 614 to enclose a plurality of radiation sources 612. Radiation sources 612 may generate a plurality of radiation beams (e.g., beamlets) through beam channels 616. The plurality of radiation beams may be configured to focus on an isocenter 210 from different directions. While each individual radiation beam may have a relatively low intensity, isocenter 210 may receive a relatively high level of radiation when multiple doses from different radiation beams accumulate at isocenter 210. In certain embodiments, isocenter 210 may correspond to a target under surgery or treatment, such as a tumor.

This disclosure describes, among other things, techniques to simulate partial patient images, such as 2D MV images or 2D CT images, as the 3D patient representation evolves during the radiation treatment, and then compare one or more actual patient images obtained during the radiation treatment to the one or more simulated partial patient images. A resulting image similarity indication can then be provided to a radiation therapy system to represent movement of the organ occurring during the radiation treatment. In this manner, the techniques of this disclosure can provide a quick and efficient way to validate the accuracy of a current 3D representation of the patient at all times during treatment, with no additional radiation to the patient.

For example, an MV image can be simulated as accurately as possible, taking all of the imaging processes into account so that the simulated MV images can provide a near-exact match to the actual MV images. The simulated MV images can be compared to measured MV images throughout the treatment. Significant differences observed during the comparison can provide an indication that the 3D patient representation is no longer representative of the actual patient anatomy, which can affect the accuracy of the treatment delivered to the patient. In such a case, the treatment can be interrupted, and a new patient representation can be generated before re-commencing the treatment. For example, a new 3D image can be acquired, or a new respiratory model can be generated that relates partial patient data to 3D image representations.

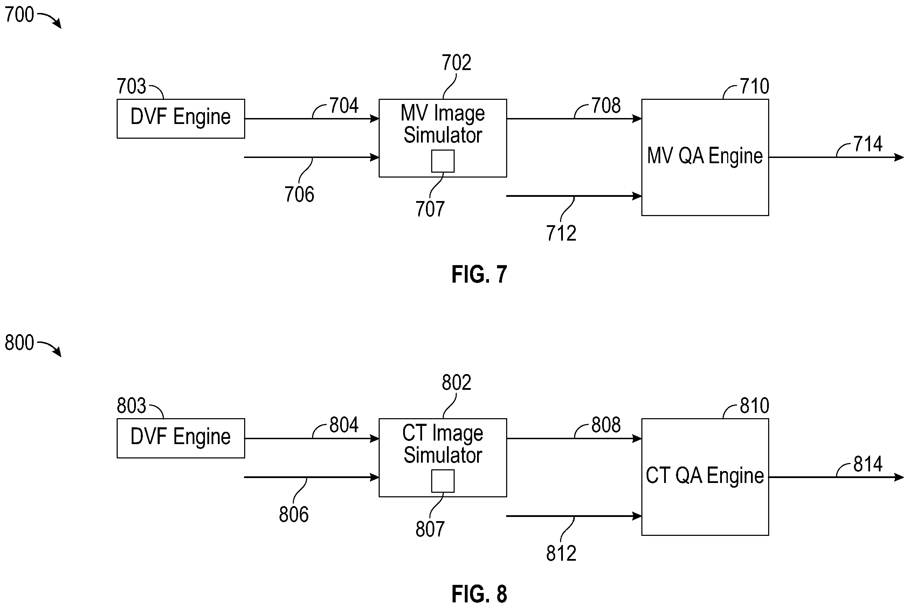

FIG. 7 is a conceptual diagram illustrating a non-limiting example of a computer-implemented method for image-guided radiotherapy that can accommodate movement of an organ of a patient during radiation treatment using various techniques of this disclosure. In the example 700 shown in FIG. 7, an MV image simulator 702 can receive simulation inputs including patient representation information 704 and radiation treatment parameter information 706.

In some examples, the patient representation information 704 can include data representing a 3D deformation vector field (DVF), which allows one to deform a previous reference image to be representative of the current time. As an example, a DVF engine 703 can determine DVFs and then use the DVFs to warp images of the patient in order to determine a patient representation information 704 that defines a patient representation.

In some examples, DVFs can be determined using grayscale information, such as without using any features. In some examples, the deformation can be a pixel-to-pixel (or voxel-to-voxel) deformation in time where each pixel (or voxel) can have a deformation vector that defines its movement from one 3D image to the next 3D image. If there is no deformation, all pixel (or voxel) deformation vectors are null. If there is deformation, the pixel (or voxel) deformation vectors point in various directions.

The MV image simulator 702 can be used to update simulated MV images, for example, as both the patient representation information 704 and the radiation treatment parameter information 706 are updated. The patient representation information 704 can include a 3D model of the patient at any given time. The treatment parameters can specify the treatment beam delivery of the linear accelerator (linac), such as MLC positions, a gantry angle, a radiation treatment beam angle, etc.

The MV image simulator 702 can generate a simulation model 707 using the 3D patient representation information 704. For example, the MV image simulator 702 can use the radiation treatment parameter information 706 of the radiation beam 208 of FIG. 2A, such as an MV treatment beam, and use the patient representation information 704 to generate a simulation model 707. Then, the MV image simulator 702 can use the simulation model 707 to determine what a partial MV image would look like based on the physics using the patient representation information 704. The MV image simulator 702 can then generate a simulated MV image 708, where the simulated MV image 708 is a partial image, such as a 2D slice or a 2D projection and not a full 3D image.

In this disclosure, a "partial image" refers to measurements acquired during the treatment itself that are not 3D images, including: 1) 2D images of a patient, such as 2D slices and 2D projections; 2) surface images of the patient; 3) measurements, such as k-space data, cardiac sensor measurements, and respiratory sensor measurements; and 4) points on the surface of a patient. The measurements are acquired sufficiently quickly for the relevant motion that is being monitored. For example, for respiration measurements should be acquired in less than 500 milliseconds (ms), such as less than 100 ms.

In some examples, the MV image simulator 702 can consider the entire imaging chain in order to generate simulated MV images. That is, both the patient and the detector can be included in the modeling in order to generate simulated MV images. In the case of phosphor-based flat panel imagers, which is an example of the imaging detector 214 of FIG. 2A, a dose calculation technique can be used by the simulation model 707 to model the transport of x-ray photons through the patient and the metal front plate, such as for calculating energy deposition events as a function of spatial position in the phosphor layer.

For example, a Monte Carlo or a Boltzmann solver simulation can be used. In a Monte Carlo simulation, for example, the random interactions of a large number of x-ray photons with the treatment head, the patient, the metal front plate and the phosphor detector are simulated. The interactions can include secondary particles generated along the path, such as electrons, as well as their subsequent interactions with the materials. Energy deposition events in the phosphor layer can (in the same step or a later step) be used to generate a number of optical photons, proportionally to the energy deposited. The random paths of the optical photons can then also be simulated until they reach the exit side of the phosphor layer. The events be recorded as a function of position, for example by separating the phosphor layer into a number of pixels, and recording the events separately in each pixel.

The effects of TFT pixels, such as noise, can also be simulated. The end result, after sufficient photon histories have been accumulated, is a 2D image that models what an actual image should look like when the patient has the expected 3D anatomical representation.

An artificial intelligence (AI) model can also be trained and used to estimate portal images, instead of using a full simulation. The AI model can be a deep leaming model built using a neural network approach, which in this example can use training sets of simulated MV image/3D image pairs generated by the previously mentioned approaches over many patients. These pairs can then be used to train a network such as a U-Net architecture. Once the model is trained, it can be used to estimate an MV image from a given 3D image.

An MV Quality Analysis (QA) engine 710 can receive and compare one or more simulated MV images 708 against one or more corresponding actual MV images 712, where the actual MV images 712 include MV images obtained in response to a radiation treatment beam providing the radiation treatment. The actual MV images 712 can be acquired by the image acquisition device 132 of FIG. 1, for example. In some examples, the actual MV images include the MV images acquired using an electronic portal imaging device (EPID), such as the imaging detector 214 of FIG. 2A.

The MV QA engine 710 can compare the simulated MV image 708 and the actual MV image 712 and determine whether they sufficiently match, and generate an image similarity indication in response, such as a pass/fail signal 714. That is, the MV QA engine 710 can compare one or more actual images obtained during the radiation treatment, to the one or more simulated partial images, and generate the resulting image similarity indication, such as the pass/fail signal 714, to represent movement of the organ occurring during the radiation treatment. The image similarity indication can validate the accuracy of a current 3D representation of the patient at all times during treatment, with no additional radiation to the patient.

The QA image processing criteria for determining whether a simulated MV image 708 and an actual MV image 712 sufficiently match in order to generate the pass/fail signal 714 can include one or more of: performing an image registration of the two images, performing a gamma analysis, performing an average pixel difference, using AI classifiers, and/or any other technique to quantify or otherwise determine a degree of similarity of images. Typically, the gamma analysis technique can be used to calculate differences between images because it incorporates into one parameter simultaneously both spatial misalignment between imaging features and percentage dose differences between the images. Thresholds on image differences can include a time tolerance, where short-lived discrepancies can be tolerated as long as they are minor relative to the length of treatment. Gamma analysis is described in "Challenges in calculation of the gamma index in radiotherapy-towards good practice," Physica Medica 36 (2017): 1-11, by Mohammad Hussein, C. H. Clark, and Andrew Nisbet.

If the pass/fail signal 714 indicates a "pass", then the radiation therapy device 202 can continue delivering the treatment to the patient. In some examples, if the pass/fail signal 714 indicates "fail", then the treatment can be stopped and the previous model generated by the MV image simulator 702 can be retrained, such as during the treatment session. For example, the radiation therapy device 202 can obtain new or updated patient representation information 704 of the patient and the MV image simulator 702 can generate a new model using the new patient representation information 704. In other examples, the model can be a population-based model.

The DVF engine 703 as well as the MV image simulator 702 and the MV QA engine 710 can be implemented fully or partially in software and/or firmware. This software and/or firmware can take the form of instructions contained in or on a non-transitory computer-readable storage medium. Those instructions can then be read and executed by one or more processors to enable performance of the operations described herein. The instructions are in any suitable form, such as but not limited to source code, compiled code, interpreted code, executable code, static code, dynamic code, and the like. Such a computer-readable medium can include any tangible non-transitory medium for storing information in a form readable by one or more computers, such as but not limited to read only memory (ROM); random access memory (RAM); magnetic disk storage media; optical storage media; flash memory; etc.

For real-time applications, there can be differences in update frequency for the various streams of data. For example, the stream of patient representation information 704 can update more slowly than the stream data representing the radiation treatment parameter information 706, and the simulated MV images may be updated more slowly than the actual MV images. This can be tolerable in some cases, as long as the lag between the different streams is relatively small compared to the whole length of treatment. In some cases, it can be advantageous to use one or more prediction algorithms to help ensure that the streams are temporally aligned or synchronized in time. For example, when a new actual MV image is available, and the simulated MV image has not been updated for 200 milliseconds (ms), for example, a simulated MV image at the current time can be predicted. Example prediction algorithms can include support vector machines, kernel density estimation, and neural networks. In some examples, an average lag time can be used in order to predict the simulated MV image.

The present techniques can be useful, such as in dynamic adaptive radiotherapy in which a 3D image can be estimated from fast partial information in combination with a model such as can provide simulation information.

Although the techniques of this disclosure have been described with respect to imaging, the present techniques can be applicable to other imaging modalities as well. For example, some linacs can have an integrated diagnostic computed tomography (CT) imager. As an example, the image acquisition device 132 can be an integrated linac and CT imaging device, such as a diagnostic CT imaging device. The diagnostic CT imaging device can be used to acquire 3D CT images during treatment such as by shifting the couch (supporting the patient) during acquisition but the diagnostic CT imaging device is not necessarily able to acquire full 3D CT images during treatment itself since the couch is generally fixed during this time.

The CT imaging device can be configured to acquire axial slices in real-time during the treatment. Axial slices, however, are generally not the most informative slice to capture respiratory motion. Most internal motion occurs in the superior/inferior direction. Axial slices have the worst resolution in this direction due to finite slice thickness. Furthermore, anatomy weaves in and out of an axial slice during respiratory motion, seemingly resulting in in-plane deformations, making it difficult to use an axial slice to detect target motion. The present techniques can be useful to model the axial CT slice image, including slice thickness effects, and to compare this modelled CT axial slice to the measured CT axial slice, such as in a similar manner as described above with respect to MV imaging above.

FIG. 8 is a conceptual diagram illustrating another non-limiting example of a computer-implemented method for image-guided radiotherapy that can accommodate movement of an organ of a patient during radiation treatment using various techniques of this disclosure. In the example 800 shown in FIG. 8, a CT image simulator 802 can receive simulation inputs including patient representation information 804 and radiation treatment parameter information 806.

In some examples, the patient representation information 804 can include data representing a 3D DVF, which allows one to deform a previous reference image to be representative of the current time. As an example, a DVF engine 803 can determine DVFs and then use the DVFs to warp images of the patient in order to determine patient representation information 804 that defines a patient representation.

In some examples, DVFs can be determined using grayscale information, such as without using any features. In some examples, the deformation can be a pixel-to-pixel (or voxel-to-voxel) deformation in time where each pixel (or voxel) can have a deformation vector that defines its movement from one 3D image to the next 3D image. If there is no deformation, all pixel (or voxel) deformation vectors are null. If there is deformation, the pixel (or voxel) deformation vectors point in various directions.

The CT image simulator 802 can be used to update simulated 2D CT image slices, for example, as both the patient representation information 804 and the radiation treatment parameter information 806 are updated. The patient representation information 804 can include a 3D model of the patient at any given time. The treatment parameters can specify the treatment beam delivery of the linear accelerator (linac), such as MLC positions, a gantry angle, a radiation treatment beam angle, etc.

The CT image simulator 802 can generate a simulation model 807 using the patient representation information 804, e.g., 3D patient representation information. For example, the CT image simulator 802 can use the radiation treatment parameter information 806 of the radiation beam 208 of FIG. 2A and use the patient representation information 804 to generate a simulation model 807. Then, the CT image simulator 802 can use the simulation model 807 to determine what a partial CT image, e.g., 2D CT image slice, would look like based on the physics using the patient representation information 804. The CT image simulator 802 can then generate a simulated CT image 808, where the simulated CT image 808 is a partial image, such as a 2D CT slice or a 2D CT projection and not a full 3D image.

In some examples, the CT image simulator 802 can consider the entire imaging chain in order to generate simulated CT images. The chain can be similar to the chain described above with respect to the MV image simulator, except that x-rays will have lower energies (kV range rather than MV range), and there likely would not be a metal plate above the phosphor. Specific details of the construction, thicknesses, choice of materials, etc. can vary but the principle is the same.

A CT Quality Analysis (QA) engine 810 can receive and compare one or more simulated CT images 808 against one or more corresponding actual CT images 812, where the actual CT images 812 include CT images obtained in response to a radiation treatment beam providing the radiation treatment. The actual CT images 812 can be acquired by the image acquisition device 132 of FIG. 1, for example.

The CT QA engine 810 can compare the simulated 2D CT image 808 and the actual 2D CT image 812 and determine whether they sufficiently match and generate a pass/fail signal 814 in response. That is, the CT QA engine 810 can compare one or more actual images obtained during the radiation treatment, to the one or more simulated partial images, and generate a resulting image similarity indication, such as the pass/fail signal 814 of FIG. 8, to represent movement of the organ occurring during the radiation treatment. The image similarity indication can validate the accuracy of a current 3D representation of the patient at all times during treatment, with no additional radiation to the patient.

The QA image processing criteria for determining whether a simulated CT image 808 and an actual CT image 812 sufficiently match in order to generate the pass/fail signal 814 can include one or more of: performing an image registration of the two images, performing a gamma analysis, performing an average pixel difference, using AI classifiers, and/or any other technique to quantify or otherwise determine a degree of similarity of images. Thresholds on image differences can include a time tolerance, where short-lived discrepancies can be tolerated as long as they are minor relative to the length of treatment.

If the pass/fail signal 814 indicates a "pass", then the radiation therapy device 202 can continue delivering the treatment to the patient. In some examples, if the pass/fail signal 814 indicates "fail", then the treatment can be stopped and the previous model generated by the CT image simulator 802 can be retrained, such as during the treatment session. For example, the radiation therapy device 202 can obtain new or updated patient representation information 804 of the patient and the CT image simulator 802 can generate a new model using the new patient representation information 704. In other examples, the model can be a population-based model.

The DVF engine 803 as well as the CT image simulator 802 and the CT QA engine 810 can be implemented fully or partially in software and/or firmware. This software and/or firmware can take the form of instructions contained in or on a non-transitory computer-readable storage medium. Those instructions can then be read and executed by one or more processors to enable performance of the operations described herein. The instructions are in any suitable form, such as but not limited to source code, compiled code, interpreted code, executable code, static code, dynamic code, and the like. Such a computer-readable medium can include any tangible non-transitory medium for storing information in a form readable by one or more computers, such as but not limited to read only memory (ROM); random access memory (RAM); magnetic disk storage media; optical storage media; flash memory; etc.

For real-time applications, there can be differences in update frequency for the various streams of data. For example, the stream of patient representation information 804 can update more slowly than the stream data representing the radiation treatment parameter information 806, and the simulated CT images may be updated more slowly than the actual CT images. This can be tolerable in some cases, as long as the lag between the different streams is relatively small compared to the whole length of treatment. In some cases, it can be advantageous to use one or more prediction algorithms to help ensure that the streams are temporally aligned or synchronized in time. In some examples, an average lag time can be used in order to predict the simulated CT image.

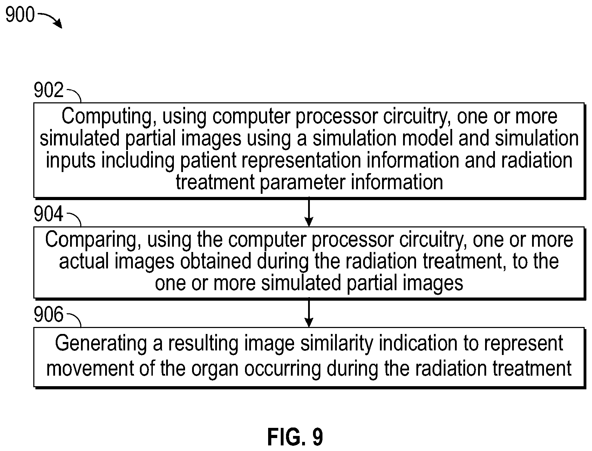

FIG. 9 illustrates a flow diagram of an example of a computer-implemented method 900 for image-guided radiotherapy that can accommodate movement of an organ of a patient during radiation treatment, according to some embodiments of the present disclosure.

At block 902, the method 900 can include computing, using computer processor circuitry, one or more simulated partial images using a simulation model and simulation inputs including patient representation information and radiation treatment parameter information. For example, an image processing device, such as the image processing device 112 of FIG. 1, can compute one or more simulated partial images, e.g., 2D Megavolt (MV) or 2D diagnostic computed tomography (CT) images.

The image processing device can compute the one or more simulated 2D MV or CT images using a simulation model, such as the simulation model 707 generated by the MV image simulator 702 of FIG. 7 or the simulation model 807 generated by the CT image simulator 802 of FIG. 8, using simulation inputs. Simulation inputs can include, for example, including patient representation information 704 and radiation treatment parameter information 706 of FIG. 7 or patient representation information 804 and radiation treatment parameter information 806 of FIG. 8.

At block 904, the method 900 can include comparing, using the computer processor circuitry, one or more actual images obtained during the radiation treatment, to the one or more simulated partial images. For example, the image processing device can compare, such as using a QA engine, a simulated image and an actual image. For example, the MV QA engine 710 of FIG. 7 can compare the simulated MV image 708 and the actual MV image 712. As another example, the CT QA engine 810 of FIG. 8 can compare the simulated CT image 808 and the actual CT image 812.

At block 906, the method 900 can include generating a resulting image similarity indication to represent movement of the organ occurring during the radiation treatment. For example, the image processing device can generate, such as using an MV QA engine, a pass/fail signal that represents movement of the organ occurring during the radiation treatment. For example, the MV QA engine 710 of FIG. 7 can determine whether the simulated MV image 708 and the actual MV image 712 sufficiently match and generate a pass/fail signal 714. In some examples, the MV QA engine can perform a gamma analysis to determine a degree of similarity of images. In other examples, a CT QA engine, such as the CT QA engine 810 of FIG. 8 can generated a pass/fail signal that represents movement of the organ occurring during the radiation treatment. The image similarity indication, e.g., a pass/fail signal, can validate the accuracy of a current 3D representation of the patient at all times during treatment, with no additional radiation to the patient.

In some examples, the actual images can include MV images obtained in response to a radiation treatment beam providing the radiation treatment. The actual images can include the MV images acquired using an electronic portal imaging device (EPID).

In some examples, the actual images can include the MV images acquired using a phosphor-based flat panel imager. In some examples, the method 900 can include modeling a transport of x-ray photons through the patient and the phosphor-based flat panel imager to calculate energy deposition events as a function of a spatial position in a phosphor layer of the phosphor-based flat panel imager. Modeling the transport of x-ray photons can include using a Monte Carlo or a Boltzmann solver simulation.

In some examples, the actual images can include diagnostic computed tomography (CT) images, such as described above with respect to FIG. 8.

In some examples, the radiation treatment parameter information includes at least one of a gantry angle, a radiation treatment beam angle, or multi-leaf collimator leaf positions.

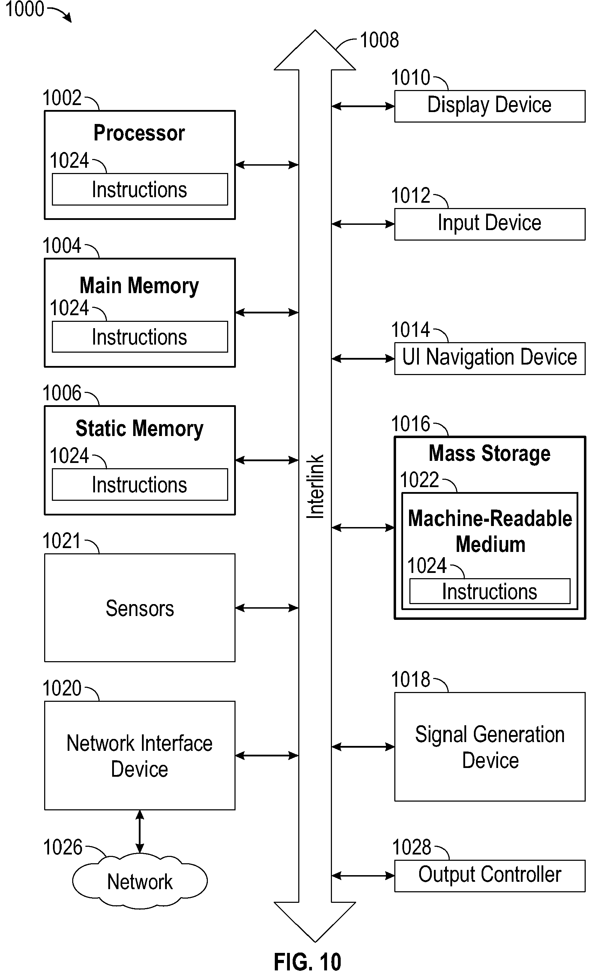

FIG. 10 illustrates a block diagram of an embodiment of a machine 1000 on which one or more of the methods as discussed herein can be implemented. In one or more embodiments, one or more items of the image processing device 112 can be implemented by the machine 1000. In alternative embodiments, the machine 1000 operates as a standalone device or may be connected (e.g., networked) to other machines. In one or more embodiments, the image processing device 112 can include one or more of the items of the machine 1000. In a networked deployment, the machine 1000 may operate in the capacity of a server or a client machine in server-client network environment, or as a peer machine in a peer-to-peer (or distributed) network environment. The machine 1000 may be a personal computer (PC), a tablet PC, a set-top box (STB), a Personal Digital Assistant (PDA), a cellular telephone, a web appliance, a network router, switch or bridge, or any machine capable of executing instructions (sequential or otherwise) that specify actions to be taken by that machine. Further, while only a single machine 1000 is illustrated, the term "machine" shall also be taken to include any collection of machines that individually or jointly execute a set (or multiple sets) of instructions to perform any one or more of the methodologies discussed herein.

The example machine 1000 includes processing circuitry (e.g., the processor 1002, a CPU, a GPU, an ASIC, circuitry, such as one or more transistors, resistors, capacitors, inductors, diodes, logic gates, multiplexers, buffers, modulators, demodulators, radios (e.g., transmit or receive radios or transceivers), sensors 1021 (e.g., a transducer that converts one form of energy (e.g., light, heat, electrical, mechanical, or other energy) to another form of energy), or the like, or a combination thereof), a main memory 1004 and a static memory 1006, which communicate with each other via a bus 1008. The machine 1000 (e.g., computer system) may further include a video display unit 1010 (e.g., a liquid crystal display (LCD) or a cathode ray tube (CRT)). The machine 1000 also includes an alphanumeric input device 1012 (e.g., a keyboard), a user interface (UI) navigation device 1014 (e.g., a mouse), a disk drive or mass storage unit 1016, a signal generation device 1018 (e.g., a speaker), and a network interface device 1020.

The disk drive or mass storage unit 1016 includes a machine-readable medium 1022 on which is stored one or more sets of data structures and instructions (e.g., software) 1024 embodying or utilized by any one or more of the methodologies or functions described herein. The instructions 1024 may also reside, completely or at least partially, within the main memory 1004 and/or within the processor 1002 during execution thereof by the machine 1000, the main memory 1004 and the processor 1002 also constituting machine-readable media.

The machine 1000 as illustrated includes an output controller 1028. The output controller 1028 manages data flow to/from the machine 1000. The output controller 1028 is sometimes called a device controller, with software that directly interacts with the output controller 1028 being called a device driver.

While the machine-readable medium 1022 is shown in an embodiment to be a single medium, the term "machine-readable medium" may include a single medium or multiple media (e.g., a centralized or distributed database, and/or associated caches and servers) that store the one or more instructions 1024 or data structures. The term "machine-readable medium" shall also be taken to include any tangible medium that is capable of storing, encoding or carrying instructions for execution by the machine and that cause the machine to perform any one or more of the methodologies of the present disclosure, or that is capable of storing, encoding or carrying data structures utilized by or associated with such instructions. The term "machine-readable medium" shall accordingly be taken to include, but not be limited to, solid-state memories, and optical and magnetic media. Specific examples of machine-readable media include non-volatile memory, including by way of example semiconductor memory devices, e.g., Erasable Programmable Read-Only Memory (EPROM), EEPROM, and flash memory devices; magnetic disks such as internal hard disks and removable disks; magneto-optical disks; and CD-ROM and DVD-ROM disks.

The instructions 1024 may further be transmitted or received over a communications network 1026 using a transmission medium. The instructions 1024 may be transmitted using the network interface device 1020 and any one of a number of well-known transfer protocols (e.g., HTTP). Examples of communication networks include a LAN, a WAN, the Internet, mobile telephone networks, Plain Old Telephone (POTS) networks, and wireless data networks (e.g., WiFi and WiMax networks). The term "transmission medium" shall be taken to include any intangible medium that is capable of storing, encoding or carrying instructions for execution by the machine, and includes digital or analog communications signals or other intangible media to facilitate communication of such software.

As used herein, "communicatively coupled between" means that the entities on either of the coupling must communicate through an item therebetween and that those entities cannot communicate with each other without communicating through the item.

### Additional Notes

The above detailed description includes references to the accompanying drawings, which form a part of the detailed description. The drawings show, by way of illustration but not by way of limitation, specific embodiments in which the disclosure can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a," "an," "the," and "said" are used when introducing elements of aspects of the disclosure or in the embodiments thereof, as is common in patent documents, to include one or more than one or more of the elements, independent of any other instances or usages of "at least one" or "one or more." In this document, the term "or" is used to refer to a nonexclusive or, such that "A or B" includes "A but not B," "B but not A," and "A and B," unless otherwise indicated.

In the appended claims, the terms "including" and "in which" are used as the plain-English equivalents of the respective terms "comprising" and "wherein." Also, in the following claims, the terms "comprising," "including," and "having" are intended to be open-ended to mean that there may be additional elements other than the listed elements, such that elements after such a term (e.g., comprising, including, having) in a claim are still deemed to fall within the scope of that claim. Moreover, in the following claims, the terms "first," "second," and "third," and so forth, are used merely as labels, and are not intended to impose numerical requirements on their objects.

Embodiments of the disclosure may be implemented with computer-executable instructions. The computer-executable instructions (e.g., software code) may be organized into one or more computer-executable components or modules. Aspects of the disclosure may be implemented with any number and organization of such components or modules. For example, aspects of the disclosure are not limited to the specific computer-executable instructions or the specific components or modules illustrated in the figures and described herein. Other embodiments of the disclosure may include different computer-executable instructions or components having more or less functionality than illustrated and described herein.

Method examples (e.g., operations and functions) described herein can be machine or computer-implemented at least in part (e.g., implemented as software code or instructions). Some examples can include a computer-readable medium or machine-readable medium encoded with instructions operable to configure an electronic device to perform methods as described in the above examples. An implementation of such methods can include software code, such as microcode, assembly language code, a higher-level language code, or the like (e.g., "source code"). Such software code can include computer-readable instructions for performing various methods (e.g., "object" or "executable code"). The software code may form portions of computer program products. Software implementations of the embodiments described herein may be provided via an article of manufacture with the code or instructions stored thereon, or via a method of operating a communication interface to send data via a communication interface (e.g., wirelessly, over the internet, via satellite communications, and the like).

Further, the software code may be tangibly stored on one or more volatile or non-volatile computer-readable storage media during execution or at other times. These computer-readable storage media may include any mechanism that stores information in a form accessible by a machine (e.g., computing device, electronic system, and the like), such as, but are not limited to, floppy disks, hard disks, removable magnetic disks, any form of magnetic disk storage media, CD-ROMS, magnetic-optical disks, removable optical disks (e.g., compact disks and digital video disks), flash memory devices, magnetic cassettes, memory cards or sticks (e.g., secure digital cards), RAMs (e.g., CMOS RAM and the like), recordable/non-recordable media (e.g., ROMs), EPROMS, EEPROMS, or any type of media suitable for storing electronic instructions, and the like. Such computer-readable storage medium coupled to a computer system bus may be accessible by the processor and other parts of the OIS.

In an embodiment, the computer-readable storage medium may have encoded a data structure for a treatment planning, wherein the treatment plan may be adaptive. The data structure for the computer-readable storage medium may be at least one of a Digital Imaging and Communications in Medicine (DICOM) format, an extended DICOM format, an XML format, and the like. DICOM is an international communications standard that defines the format used to transfer medical image-related data between various types of medical equipment. DICOM RT refers to the communication standards that are specific to radiation therapy.

In various embodiments of the disclosure, the method of creating a component or module can be implemented in software, hardware, or a combination thereof. The methods provided by various embodiments of the present disclosure, for example, can be implemented in software by using standard programming languages such as, for example, Compute Unified Device Architecture (CUDA), C, C++, Java, Python, and the like; and using standard machine learning/deep leaming library (or API), such as tensorflow, torch and the like; and combinations thereof. As used herein, the terms "software" and "firmware" are interchangeable, and include any computer program stored in memory for execution by a computer.

A communication interface includes any mechanism that interfaces to any of a hardwired, wireless, optical, and the like, medium to communicate to another device, such as a memory bus interface, a processor bus interface, an Internet connection, a disk controller, and the like. The communication interface can be configured by providing configuration parameters and/or sending signals to prepare the communication interface to provide a data signal describing the software content. The communication interface can be accessed via one or more commands or signals sent to the communication interface.

The present disclosure also relates to a system for performing the operations herein. This system may be specially constructed for the required purposes, or it may comprise a general-purpose computer selectively activated or reconfigured by a computer program stored in the computer. The order of execution or performance of the operations in embodiments of the disclosure illustrated and described herein is not essential, unless otherwise specified. That is, the operations may be performed in any order, unless otherwise specified, and embodiments of the disclosure may include additional or fewer operations than those disclosed herein. For example, it is contemplated that executing or performing a particular operation before, contemporaneously with, or after another operation is within the scope of aspects of the disclosure.

In view of the above, it will be seen that the several objects of the disclosure are achieved, and other beneficial results attained. Having described aspects of the disclosure in detail, it will be apparent that modifications and variations are possible without departing from the scope of aspects of the disclosure as defined in the appended claims. As various changes could be made in the above constructions, products, and methods without departing from the scope of aspects of the disclosure, it is intended that all matter contained in the above description and shown in the accompanying drawings shall be interpreted as illustrative and not in a limiting sense.

The above description is intended to be illustrative, and not restrictive. For example, the above-described examples (or one or more aspects thereof) may be used in combination with each other. **In** addition, many modifications may be made to adapt a particular situation or material to the teachings of the disclosure without departing from its scope. While the dimensions, types of materials and coatings described herein are intended to define the parameters of the disclosure, they are by no means limiting and are example embodiments. Many other embodiments will be apparent to those of skill in the art upon reviewing the above description. The scope of the disclosure should, therefore, be determined with reference to the appended claims, along with the full scope of equivalents to which such claims are entitled.

## Claims

1. A radiotherapy system that can accommodate movement of an organ of a patient during radiation treatment, the radiotherapy system comprising:
an image acquisition device configured to acquire images of an anatomical region of interest of a patient;
a radiotherapy device configured to deliver a dose of radiation to the anatomical region of interest based on the images of the anatomical region of interest; and
a processor configured to, during the radiation treatment:
compute (902), using computer processor circuitry, one or more simulated partial images using a simulation model and simulation inputs including patient representation information and radiation treatment parameter information, wherein the one or more simulated partial images simulate what an actual image should look like;
compare (904), using the computer processor circuitry, one or more actual images obtained during the radiation treatment, to the one or more simulated partial images;
generate (906), using the computer processor circuitry, a resulting image similarity indication to represent the movement of the organ occurring during the radiation treatment;
generate a fail signal (714, 814) to stop the radiation treatment if it is determined, based on the image similarity indication, that the one or more actual images do not sufficiently match the one or more simulated partial images; and
provide the fail signal to the radiotherapy device to stop the radiation treatment.

2. The system of claim 1, wherein the one or more actual images include MV images obtained in response to a radiation treatment beam providing the radiation treatment.

3. The system of claim 2, wherein the one or more actual images include the MV images acquired using an electronic portal imaging device, EPID.

4. The system of claim 2, wherein the one or more actual images include the MV images acquired using a phosphor-based flat panel imager.

5. The system of claim 4, the processor being further configured to:
model a transport of x-ray photons through the patient and the phosphor-based flat panel imager to calculate energy deposition events as a function of a spatial position in a phosphor layer of the phosphor-based flat panel imager.

6. The system of claim 5, wherein modeling the transport of x-ray photons includes:
using a Monte Carlo or a Boltzmann solver simulation.

7. The system of claim 1, wherein the one or more actual images include diagnostic computed tomography, CT, images.

8. The system of claim 1, wherein the radiation treatment parameter information includes at least one of a gantry angle, a radiation treatment beam angle, or multi-leaf collimator leaf positions.

9. The system of claim 1, wherein comparing, using the computer processor circuitry, the one or more actual images obtained during the radiation treatment, to the one or more simulated partial images includes:
performing a gamma analysis to determine a degree of similarity of images.

10. The system of claim 1, the processor being further configured to:
obtain updated patient representation information based on the resulting image similarity indication.

11. The system of claim 1, wherein the one or more simulated partial images include 2D images.

12. The system of claim 1, wherein the one or more simulated partial images include at least one of one or more simulated 2D Megavolt, MV, images or 2D diagnostic computed tomography, CT, images.

13. The system of any preceding claim, wherein the image similarity indication enables validation of the accuracy of the patient representation.

14. The system of any preceding claim, wherein the one or more simulated partial images are updated in real-time during the radiation treatment.

15. A computer-readable medium configured to include instructions to perform functions during radiation treatment, the functions comprising :
computing (902), using computer processor circuitry, one or more simulated partial images using a simulation model and simulation inputs including patient representation information and radiation treatment parameter information, wherein the one or more simulated partial images simulate what an actual image should look like;
comparing (904), using the computer processor circuitry, one or more actual images obtained during the radiation treatment, to the one or more simulated partial images;
generating (906), using the computer processor circuitry, a resulting image similarity indication to represent the movement of the organ occurring during the radiation treatment;
generate a fail signal (714, 814) to stop the radiation treatment if it is determined, based on the image similarity indication, that the one or more actual images do not sufficiently match the one or more simulated partial images; and
provide the fail signal to a radiotherapy device to stop the radiation treatment.

## Patentansprüche

1. Strahlentherapiesystem, das Bewegung eines Organs eines Patienten während Strahlenbehandlung berücksichtigen kann, wobei das Strahlentherapiesystem umfasst:
eine Bilderfassungsvorrichtung, dazu ausgelegt, Bilder einer anatomischen Region von Interesse eines Patienten zu erfassen;
eine Strahlentherapievorrichtung, dazu ausgelegt, eine Strahlendosis auf der Grundlage der Bildern der anatomischen Region von Interesse an die anatomische Region von Interesse abzugeben; und
einen Prozessor, dazu ausgelegt, während der Strahlenbehandlung:
Berechnen (902), unter Verwendung einer Computerprozessorschaltung, eines oder mehrerer simulierter Teilbilder unter Verwendung eines Simulationsmodells und von Simulationseingaben, einschließlich Patientendarstellungsinformationen und Strahlenbehandlungsparameterinformationen, wobei das eine oder die mehreren simulierten Teilbilder simulieren, wie ein tatsächliches Bild aussehen sollte;
Vergleichen (904), unter Verwendung der Computerprozessorschaltung, eines oder mehrerer tatsächlicher Bilder, die während der Strahlenbehandlung erhalten worden sind, mit dem einen oder den mehreren simulierten Teilbildern;
Erzeugen (906), unter Verwendung der Computerprozessorschaltung, einer erhaltenen Bildähnlichkeitsanzeige, um die Bewegung des Organs, die während der Strahlenbehandlung auftritt, darzustellen;
Erzeugen eines Fehlersignals (714, 814), um die Strahlenbehandlung zu stoppen, wenn auf der Grundlage der Bildähnlichkeitsanzeige bestimmt wird, dass das eine oder die mehreren tatsächlichen Bilder nicht ausreichend mit dem einen oder den mehreren simulierten Teilbildern übereinstimmen; und
Ausgeben des Fehlersignals an die Strahlentherapievorrichtung, um die Strahlenbehandlung zu stoppen.

2. System nach Anspruch 1, wobei das eine oder die mehreren tatsächlichen Bilder MV-Bilder umfassen, die als Reaktion auf einen Strahlenbehandlungsstrahl erhalten werden, der die Strahlenbehandlung bereitstellt.

3. System nach Anspruch 2, wobei das eine oder die mehreren tatsächlichen Bilder die MV-Bilder umfassen, die unter Verwendung einer elektronischen Portalbildgebungsvorrichtung, EPID, erfasst werden.

4. System nach Anspruch 2, wobei das eine oder die mehreren tatsächlichen Bilder die MV-Bilder umfassen, die unter Verwendung eines Flachbildgebers auf Leuchtstoffbasis erfasst werden.

5. System nach Anspruch 4, wobei der Prozessor ferner ausgelegt ist zum:
Modellieren eines Transports von Röntgenphotonen durch den Patienten und den Flachbildgeber auf Leuchtstoffbasis, um Energiedepositionsereignisse als Funktion einer räumlichen Position in einer Leuchtstoffschicht des Flachbildgebers auf Leuchtstoffbasis zu berechnen.

6. System nach Anspruch 5, wobei Modellieren des Transports von Röntgenphotonen umfasst:
Verwenden einer Monte-Carlo- oder Boltzmann-Solver-Simulation.

7. System nach Anspruch 1, wobei das eine oder die mehreren tatsächlichen Bilder diagnostische Computertomographie-, CT-, Bilder umfassen.

8. System nach Anspruch 1, wobei die Strahlenbehandlungsparameterinformationen wenigstens eines von einem Gantrywinkel, einem Strahlenbehandlungsstrahlwinkel und Multilamellenkollimator-Lamellenpositionen umfassen.

9. System nach Anspruch 1, wobei Vergleichen, unter Verwendung der Computerprozessorschaltung, des einen oder der mehreren tatsächlichen Bilder, die während der Strahlenbehandlung erhalten worden sind, mit dem einen oder den mehreren simulierten Teilbildern umfasst:
Durchführen einer Gamma-Analyse, um einen Ähnlichkeitsgrad von Bildern zu bestimmen.

10. System nach Anspruch 1, wobei der Prozessor ferner ausgelegt ist zum:
Erhalten aktualisierter Patientendarstellungsinformationen auf der Grundlage der erhaltenen Bildähnlichkeitsanzeige.

11. System nach Anspruch 1, wobei das eine oder die mehreren simulierten Teilbilder 2D-Bilder umfassen.

12. System nach Anspruch 1, wobei das eine oder die mehreren simulierten Teilbilder wenigstens eines von einem oder mehreren simulierten 2D-Megavolt-, MV-, Bildern oder 2D-diagnostischen Computertomographie-, CT-, Bildern umfassen.

13. System nach einem der vorstehenden Ansprüche, wobei die Bildähnlichkeitsanzeige Validierung der Genauigkeit der Patientendarstellung ermöglicht.

14. System nach einem der vorstehenden Ansprüche, wobei das eine oder die mehreren simulierten Teilbilder während der Strahlenbehandlung in Echtzeit aktualisiert werden.

15. Computerlesbares Medium, dazu ausgelegt, Anweisungen zur Durchführung von Funktionen während Strahlenbehandlung zu umfassen, wobei die Funktionen umfassen:
Berechnen (902), unter Verwendung einer Computerprozessorschaltung, eines oder mehrerer simulierter Teilbilder unter Verwendung eines Simulationsmodells und von Simulationseingaben, einschließlich Patientendarstellungsinformationen und Strahlenbehandlungsparameterinformationen, wobei das eine oder die mehreren simulierten Teilbilder simulieren, wie ein tatsächliches Bild aussehen sollte;
Vergleichen (904), unter Verwendung der Computerprozessorschaltung, eines oder mehrerer tatsächlicher Bilder, die während der Strahlenbehandlung erhalten worden sind, mit dem einen oder den mehreren simulierten Teilbildern;
Erzeugen (906), unter Verwendung der Computerprozessorschaltung, einer resultierenden Bildähnlichkeitsanzeige, um die Bewegung des Organs, die während der Strahlenbehandlung auftritt, darzustellen;
Erzeugen eines Fehlersignals (714, 814), um die Strahlenbehandlung zu stoppen, wenn auf der Grundlage der Bildähnlichkeitsanzeige bestimmt wird, dass das eine oder die mehreren tatsächlichen Bilder nicht ausreichend mit dem einen oder den mehreren simulierten Teilbildern übereinstimmen; und
Ausgeben des Fehlersignals an eine Strahlentherapievorrichtung, um die Strahlenbehandlung zu stoppen.

## Revendications

1. Système de radiothérapie pouvant tenir compte du mouvement d'un organe d'un patient pendant un traitement par rayonnement, le système de radiothérapie comprenant :
un dispositif d'acquisition d'images configuré pour acquérir des images d'une région anatomique d'intérêt d'un patient ;
un dispositif de radiothérapie configuré pour administrer une dose de rayonnement à la région anatomique d'intérêt sur la base des images de la région anatomique d'intérêt ; et
un processeur configuré pour, pendant le traitement par rayonnement :
calculer (902), au moyen d'une circuiterie de processeur informatique, une ou plusieurs images partielles simulées au moyen d'un modèle de simulation et d'entrées de simulation comportant des informations de représentation de patient et des informations de paramètres de traitement par rayonnement, la ou les images partielles simulées simulant l'apparence que devrait avoir une image réelle ;
comparer (904), au moyen de la circuiterie de processeur informatique, une ou plusieurs images réelles obtenues pendant le traitement par rayonnement à la ou aux images partielles simulées ;
générer (906), au moyen de la circuiterie de processeur informatique, une indication résultante de similarité d'images afin de représenter le mouvement de l'organe survenant pendant le traitement par rayonnement ;
générer un signal d'échec (714, 814) afin d'interrompre le traitement par rayonnement s'il est déterminé, sur la base de l'indication de similarité d'images, que la ou les images réelles ne correspondent pas suffisamment à la ou aux images partielles simulées ; et
fournir le signal d'échec au dispositif de radiothérapie afin d'interrompre le traitement par rayonnement.

2. Système selon la revendication 1, dans lequel la ou les images réelles comportent des images MV obtenues en réponse à un faisceau de traitement par rayonnement fournissant le traitement par rayonnement.

3. Système selon la revendication 2, dans lequel la ou les images réelles comportent les images MV acquises au moyen d'un dispositif électronique d'imagerie portale, EPID.

4. Système selon la revendication 2, dans lequel la ou les images réelles comportent les images MV acquises au moyen d'un imageur à panneau plat à base de phosphore.

5. Système selon la revendication 4, le processeur étant en outre configuré pour :
modéliser un transport de photons de rayons X à travers le patient et l'imageur à panneau plat à base de phosphore afin de calculer des événements de dépôt d'énergie en fonction d'une position spatiale dans une couche de phosphore de l'imageur à panneau plat à base de phosphore.

6. Système selon la revendication 5, dans lequel la modélisation du transport de photons de rayons X comporte :
l'utilisation d'une simulation de Monte Carlo ou d'une simulation par solveur de Boltzmann.

7. Système selon la revendication 1, dans lequel la ou les images réelles comportent des images de tomodensitométrie, CT, de nature diagnostique.

8. Système selon la revendication 1, dans lequel les informations de paramètres de traitement par rayonnement comportent au moins un élément choisi parmi un angle de portique, un angle de faisceau de traitement par rayonnement ou des positions de lames d'un collimateur multilame.

9. Système selon la revendication 1, dans lequel la comparaison, au moyen de la circuiterie de processeur informatique, de la ou des images réelles obtenues pendant le traitement par rayonnement à la ou aux images partielles simulées comporte :
la réalisation d'une analyse gamma afin de déterminer un degré de similarité d'images.

10. Système selon la revendication 1, le processeur étant en outre configuré pour :
obtenir des informations de représentation de patient mises à jour sur la base de l'indication résultante de similarité d'images.

11. Système selon la revendication 1, dans lequel la ou les images partielles simulées comportent des images 2D.

12. Système selon la revendication 1, dans lequel la ou les images partielles simulées comportent une ou plusieurs images 2D en mégavoltage, MV, simulées et/ou une ou plusieurs images 2D de tomodensitométrie, CT, de nature diagnostique.

13. Système selon l'une quelconque des revendications précédentes, dans lequel l'indication de similarité d'images permet une validation de la précision de la représentation de patient.

14. Système selon l'une quelconque des revendications précédentes, dans lequel la ou les images partielles simulées sont mises à jour en temps réel pendant le traitement par rayonnement.

15. Support lisible par ordinateur configuré pour comporter des instructions pour réaliser des fonctions pendant un traitement par rayonnement, les fonctions comprenant :
le calcul (902), au moyen d'une circuiterie de processeur informatique, d'une ou de plusieurs images partielles simulées au moyen d'un modèle de simulation et d'entrées de simulation comportant des informations de représentation de patient et des informations de paramètres de traitement par rayonnement, la ou les images partielles simulées simulant l'apparence que devrait avoir une image réelle ;
la comparaison (904), au moyen de la circuiterie de processeur informatique, d'une ou de plusieurs images réelles obtenues pendant le traitement par rayonnement à la ou aux images partielles simulées ;
la génération (906), au moyen de la circuiterie de processeur informatique, d'une indication résultante de similarité d'images afin de représenter le mouvement de l'organe survenant pendant le traitement par rayonnement ;
la génération d'un signal d'échec (714, 814) afin d'interrompre le traitement par rayonnement s'il est déterminé, sur la base de l'indication de similarité d'images, que la ou les images réelles ne correspondent pas suffisamment à la ou aux images partielles simulées ; et
la fourniture du signal d'échec à un dispositif de radiothérapie afin d'interrompre le traitement par rayonnement.
